# EUROPEAN PATENT SPECIFICATION

(11) **EP 3 089 736 B1**
(45) Date of publication and mention of the grant of the patent: **23.07.2025**
(21) Application number: 14876433.5
(22) Date of filing: 30.12.2014
(51) Int. Cl.: A61K 47/38, A61K 47/30, A61K 9/14

(54) **PHARMACEUTICAL COMPOSITIONS FOR POORLY WATER-SOLUBLE COMPOUNDS**
PHARMAZEUTISCHE ZUSAMMENSETZUNGEN FÜR SCHWER WASSERLÖSLICHE VERBINDUNGEN
COMPOSITIONS PHARMACEUTIQUES POUR COMPOSÉS FAIBLEMENT HYDROSOLUBLES

(30) Priority: 31.12.2013 US 201361922180 P
(43) Date of publication of application: 09.11.2016
(73) Proprietor: Ascendia Pharmaceuticals, LLC, North Brunswick, NJ 08902 (US)
(72) Inventor: HUANG, Jingjun, North Brunswick, NJ 08902 (US); TOMINAGA, Kaoru, North Brunswick, NJ 08902 (US); YU, Hui, Newtown, PA 18940 (US)
(74) Representative: Wichmann, Hendrik
(86) International application number: PCT/US2014/072704
(87) International publication number: WO 2015/103230

(56) References cited:
- WO-A1-2013/040120
- WO-A1-2013/040187
- WO-A2-03/000226
- WO-A2-2009/026461
- DE-A1- 102009 036 646
- JP-A- 2004 067 606
- US-A1- 2011 306 632
- SIX, KAREL ET AL.: "Increased physical stability and improved dissolution properties of itraconazole, a class II drug, by solid dispersions that combine fast- and slow- dissolving polymers.", JOURNAL OF PHARMACEUTICAL SCIENCES, vol. 93, no. 1, 2004, pages 124 - 131, XP001185341

## Description

### Field of the invention

The present invention relates to a pharmaceutical solid dispersion composition as defined in the claims containing at least one poorly water-soluble acidic active pharmaceutical ingredient (API), to improve API solubility throughout the Gastrointestinal (GI) tract and thus improving the bioavailability and reducing absorption variability.

### Background of the invention

Poorly water soluble APIs are problematic in pharmaceutical formulations. Without the APIs dissolving in aqueous solutions at the biological pH range, the absorption of APIs will be very variable and poor which limits the therapeutic effects of the APIs.

Solid dispersions have been demonstrated to be useful in improving drug solubility and bioavailability of poorly water soluble drugs. There have been numerous compositions and methods to prepare various forms of solid dispersions with poorly water soluble APIs. Solid dispersion of a poorly water soluble API can be prepared by dispersing the API in a polymer matrix of either a water-soluble or a pH sensitive polymer in nature to improve the aqueous solubility. In U.S. Pat. No. 5,456,923, Nakamichi et. al. disclosed a new process to manufacture solid dispersions of poorly soluble APIs with a water soluble polymer by hot melt technology; DE 10 2009 036 646 discloses a composition comprising prasugel as an API and a hydrophilic polymer, and the method for producing said composition to provide a solid dispersion.
US 2011/0306632 discloses a solid dispersion product comprising an API and a pharmaceutically acceptable water-soluble polymeric carrier, and a pharmaceutically acceptable surfactant.
WO 2013/040120 discloses pharmaceutical dosage forms of cyclopropanecarboxylic acid{2-[(1S)-1-(3-ethoxy-4-methoxy-phenyl)-2-methanesulfonyl-ethyl]-3-oxo-2,3-dihydro-1H-isoindol-4-yl}-amide.
WO 2009/026461 discloses compositions including an API and a pharmaceutically acceptable excipients and methods for making such compositions by thermokinetic compounding into a composite.
WO 03/000226 discloses polymer/drug assemblies which greatly enhance the concentration of a low-solubility drug in aqueous solution.
Miyajima et. al. in U.S. Pat. No. 4,983,593 disclosed a pharmaceutical composition of a solvate of dihydropyridine with an enteric polymer, i.e. hydroxypropylmethylcellulose acetate succinate (HPMCAS). However, solid dispersions prepared with only one polymer may encounter problems associated with dissolution of the API. For example, for solid dispersions of API with a water-soluble polymer, supersaturation of API in aqueous media caused by rapid dissolution of water-soluble polymer from the matrix may cause recrystallization of the API from the dissolution medium that reduce bioavailability. For solid dispersion of API with an enteric polymer, very low level of API dissolution in gastric fluid of low pH range, mainly due to enteric polymer nature, may delay drug absorption that cause difficulty to maintain therapeutic concentration. Besides variable API dissolution in GI fluid as a result of variation of GI fluid pH caused by food, or by patient variation may also cause variable pharmacokinetics profiles. For solid dispersions of API with a gastric-soluble polymer that is soluble at pH below 5 and insoluble at pH above 5 (such as Eudragit E), precipitation of the gastric-soluble polymer in intestine fluid of higher pH above 5 will cause variation in drug absorption/bioavailability and variable pharmacokinetics profiles.

Poorly water soluble APIs with weakly basic or weakly acidic characteristics have a pH-dependent solubility profile and can have a wide range of solubility in the gastrointestinal tract. For example, itraconazole is a weakly basic compound with a pKa (basic) of 3.7, has a solubility of 3.5 mg/mL in gastric fluid and 0.2 µg/mL in intestinal fluid, and diclofenac is a weakly acidic compound with a pKa (acidic) of 4.0, has a solubility of 1 µg/mL in gastric fluid and 1113 µg/mL (as sodium salt) in intestinal fluid of neutral pH. To increase the solubility of these APIs, poorly water soluble APIs have been dispersed into a water-soluble polymers to achieve a high API solubility in aqueous medium; or into a pH sensitive polymer, such as an enteric polymer to improve the solubility of weakly basic APIs at higher pH levels, or into a gastric-soluble polymer that is soluble at pH below 5 and insoluble at pH above 5 (e.g. Eudragit E, Chitosan) to improve the solubility of weakly acidic APIs at lower pH levels.

For those instances using water soluble polymer, high API concentration achieved by dispersion of API in water soluble polymer could lead to super-saturation of the API in gastrointestinal fluid, which may result in API recrystallization before absorption takes place in intestinal tract (Kai, et al., Chem. Pharm. Bull. 44(3) 568-571 (1996)). Moreover, pH-dependent dissolution profiles of acidic and basic compounds cannot be overcome by using water soluble polymer.

For those instances using a pH sensitive enteric polymer for a weakly basic API, even though enteric polymer may help to maintain API super-saturation in intestine fluid, drug initial dissolution at gastric fluid is delayed or depressed due to insolubility of enteric polymer at the gastric pH, which could cause a delay in drug absorption since the API's initial dissolution may not be enough to reach a therapeutic effective concentration level. Besides, drug absorption for enteric polymer dispersion may be highly variable since inter and intra-patients may have very different GI pH values at different time or before and after meal.

For those instances using a pH sensitive gastric-soluble polymer that is soluble at gastric pH and insoluble in intestine pH, initial weakly acidic API dissolution at gastric fluid may be improved by the polymer. However, insolubility of the polymer at intestine fluid could cause precipitation of drug with the polymer in intestine fluid and could have negative effect on the absorption and bioavailability of weakly acidic compound in intestine tract. Besides high API super-saturation caused by fast dissolution of the polymer at gastric pH could also lead API recrystallization before absorption takes place in intestine fluid.

Due to potential drug-polymer interaction or complex formation, some pH-sensitive polymers have proven to be useful to maintain supersaturation of neutral or non-ionizable compounds in GI fluid. However, utilizing of pH sensitive polymers such as enteric polymer or gastric-soluble polymer can cause a pH-dependent dissolution profiles of neutral/non-ionizable compounds. This may result in highly variable drug absorption profiles due to difference in GI pH between patient to patient or among different times or disease status of the same patient. Accordingly, there is an unmet need for solutions for pharmaceutical compositions for poorly water-soluble compounds.

### Summary of the Invention

The pharmaceutical compositions of this disclosure provide solution to the problems of the previously known art. The pharmaceutical compositions of the present invention differ from previous findings in that at least one water-soluble polymer and at least one pH sensitive polymer and/or pharmaceutically acceptable surfactants are combined to form a matrix of solid dispersion with poorly water soluble APIs as defined in the claims, the solubility/dissolution of which in said composition were found surprisingly to be enhanced and be relatively pH independent by this novel formulation approach. As a result, reproducible and continuous drug release throughout the GI tract physiological pH range of 1.0-8.0 may be provided by the formulations of this invention. These are very important dissolution characteristics that ensure consistent absorption of APIs in GI tract and reproducible PK profiles with a reduced food effect.

Combination of at least one water-soluble and at least one pH sensitive polymer and optionally pharmaceutically acceptable surfactants to form uniform dispersion of pH-sensitive polymer in the matrix addresses the shortcomings of previous solid dispersion formulations utilizing single polymer by means of 1) minimizing the pH sensitivity of APIs' solubility and stabilizing API solubilization in the GI fluid; and 2) reducing the pH sensitivity of polymer's solubility in the GI fluid. This unique feature in solubilization of the pH-sensitive polymer(s) (enteric polymer and gastric-soluble polymer) throughout the GI tract by dispersing these pH-sensitive polymer in water-soluble polymer(s) at certain ratios will help the pH sensitive polymer to stay solubilized/suspended in both the gastric and intestinal fluids at molecular or colloidal level, which will in turn ensure their maximum solubilization effect on the APIs in different dissolution conditions.

By combining the water-soluble and pH sensitive polymers and/or pharmaceutically acceptable surfactants, the dissolution profile of the API in solid dispersion form will be improved both in gastric fluid and intestine fluid as the water-soluble polymers solubilize/suspense the pH sensitive polymer, and the pH-sensitive polymer and/or water soluble polymer maintains the soluble status of the API in the GI tract. The pharmaceutically acceptable surfactants with an amphiphilic property can increase wetting of the API for faster dissolution and can also improve solubilization/suspension of API and the pH sensitive polymer.

### Brief description of the drawings

In the accompanying drawings:
Fig. 1 shows the results of the dissolution test of prasugrel performed using solid dispersions prepared with hydroxypropyl methyl cellulose (HPMC), at pH 1.2 (0.1N hydrochloric acid) and pH 6.8 (phosphate buffer) solutions.
Fig. 2 shows the results of the dissolution test of prasugrel performed using solid dispersions prepared with polyvinyl caprolactam-polyvinyl acetate-polyethylene glycol graft copolymer (Soluplus^{®}) respectively, at pH 1.2 (0.1N hydrochloric acid) and pH 6.8 (phosphate buffer) solutions.
Fig. 3 show the results of the dissolution test of prasugrel from the solid dispersion prepared with combination of hydroxypropyl methylcellulose acetate succinate (HPMCAS) and Soluplus^{®} at pH 1.2 and pH 6.8. The dissolution of fused amorphous prasugrel is used as the reference sample.
Fig. 4 show the result of the dissolution test of prasugrel from the solid dispersion prepared with combination of HPMC, HPMCAS and Soluplus^{®} at pH 1.2 and pH 6.8. The dissolution of fused amorphous prasugrel is used as the reference sample.
Fig. 5 shows the results of the dissolution test of clopidogrel from solid dispersions prepared with hydroxypropyl methyl cellulose (HPMC), at pH 1.2 (0.1N hydrochloric acid) and pH 6.8 (phosphate buffer) solutions.
Fig. 6 shows the result of the dissolution test of clopidogrel from solid dispersion prepared with combination of HPMC, HPMCAS and Tween 80 at pH 1.2 and pH 6.8.
Fig. 7 shows the result of the dissolution test of clopidogrel from solid dispersion prepared with combination of Eudragit EPO^{®} by Evonik at pH 1.2 and pH 6.8.
Fig. 8 shows the result of the dissolution test of clopidogrel from solid dispersion prepared with combination of Eudragit EPO^{®} by Evonik and Soluplus^{®} at pH 1.2 and pH 6.8.
Fig. 9 shows the result of the dissolution test of diclofenac from solid dispersion prepared with gastric-soluble acrylic copolymers (Eudragit EPO^{®} by Evonik) at pH 1.2 and pH 6.8.
Fig. 10 shows the result of the dissolution test of diclofenac from solid dispersion prepared with Vinylpyrrolidone-vinyl acetate copolymer (PVPVA 64 or Kollidon^{®} VA 64 by Evonik) at pH 1.2 and pH 6.8.
Fig. 11 shows the result of the dissolution test of diclofenac from solid dispersion prepared with gastric-soluble acrylic copolymers (EPO or Eudragit E^{®} by Evonik) and Vinylpyrrolidone-vinyl acetate copolymer (PVPVA 64 or Kollidon^{®} VA 64 by Evonik) at pH 1.2 and pH 6.8.
Fig. 12 shows the result of the dissolution test of diclofenac from solid dispersion prepared with enteric polymer (HPMCAS) and HPMC 603 at pH 1.2 and pH 6.8.
Fig. 13 shows the result of the dissolution test of ibuprofen from solid dispersion prepared with gastric-soluble acrylic copolymers (EPO or Eudragit E^{®} by Evonik) at pH 1.2 and pH 6.8. Dissolution of ibuprofen alone in pH 1.2 is also shown for comparison.
Fig. 14 shows the result of the dissolution test of ibuprofen from solid dispersion prepared with polyvinyl caprolactam-polyvinyl acetate-polyethylene glycol graft copolymer (Soluplus^{®} by BASF) at pH 1.2 and pH 6.8.
Fig. 15 shows the result of the dissolution test of ibuprofen from solid dispersion prepared with Eudragit E and Soluplus^{®} at pH 1.2 and pH 6.8.
Fig. 16 shows the result of the dissolution test of ibuprofen from solid dispersion prepared with Eudragit E, HPMC, and Soluplus^{®} at pH 1.2 and pH 6.8.
Fig. 17 shows the result of the dissolution test of ibuprofen from solid dispersion prepared with Eudragit E, Span 20, and Soluplus^{®} at pH 1.2 and pH 6.8.
Fig. 18 shows the result of the dissolution test of ibuprofen from solid dispersion prepared with Eudragit E, and HPMC at pH 1.2 and pH 6.8.
Fig. 19 shows the result of the dissolution test of ibuprofen from solid dispersion prepared with HPMCAS and HPMC at pH 1.2 and pH 6.8.
Fig. 20 shows the result of the dissolution test of apixaban from solid dispersion prepared with HPMC 603 at pH 1.2 and pH 6.8.
Fig. 21 shows the result of the dissolution test of apixaban from solid dispersion prepared with HPMCAS at pH 1.2 and pH 6.8.
Fig. 22 shows the result of the dissolution test of apixaban from solid dispersion prepared with HPMC 603 and HPMCAS at pH 1.2 and pH 6.8.

### Detailed description of the invention

The pharmaceutical compositions of the present invention provide a pH independent solubility and continuous dissolution profile of poorly water soluble active pharmacetutical ingredients (API) or compounds throughout the GI tract. The pharmaceutical compositions of the present invention provide solid dispersions prepared in order to improve the solubility/dissolution of API, combining water-soluble polymer(s), pH sensitive polymer(s) and/or pharmaceutical acceptable surfactant(s), wherein the API comprises an API having a solubility of no more than 1 mg/mL at pH 1.2 for weakly acidic compound, and no more than 1 mg/mL at any pH between the physiological pH of 1.0-8.0 for neutral or non-ionizable compounds

The term "solid dispersion" refer to an ingredient, small molecule or polymer, typically of less than 10 µm in diameter, dispersed in a polymeric matrix, and/or more particularly, at least an ingredient, small molecule or polymer, typically of less than 10 µm in diameter, are dispersed in at least one polymer in the solid state.

The term "active pharmaceutical ingredient" (API) can be used interchangeably with the terms "new chemical entity", "drug", "compound", "therapeutic agent", etc.

By "poorly water soluble API", it is meant that the API has less than 1 mg/mL solubility in the physiological pH range at 25 degree Celsius. The solubility of an API can be determined by adding the highest dose strength in 250 mL of aqueous solutions ranging from pH 1 to 7.4 to cover GI physiological conditions. If there is less than 250 mg of API dissolved in 250 mL of solution of any pH from 1-7.4, the API is considered to be poorly water soluble.

As used herein, the term "weakly basic compound", as well as reference to any specific new chemical entity, drug, or active pharmaceutical ingredient, includes the base, pharmaceutically acceptable salts, polymorphs, stereoisomers, solvates, esters and mixtures thereof, which is a chemical base in which protonation is incomplete in aqueous medium. In one embodiment, the weakly basic compound of the compositions of the present invention can refer to a compound having at least one pKa in the range of less than 14, wherein pKa can be measured or by calculation. In another embodiment, the weakly basic compound of the compositions of the present invention can refer to a compound having at least one pKa of less than 14, which has a pH dependent solubility between physiological pH with a lower solubility at higher pH. In another embodiment, the weakly basic drug of the compositions of the present invention can refer to a compound having at least one pKa of 0.0-10.0, which has a pH dependent solubility between physiological pH of 1.0-8.0 with a lowest solubility at around pH 6.0-8.0. In another embodiment, the weakly basic compound has a solubility of not more than about 1 mg/mL at pH 6.8. In another embodiment, the weakly basic compound includes at least one basic nitrogen atom. In yet another embodiment, the weakly basic compound has a pKa of less than 14, and a solubility of not more than about 1 mg/mL at pH 6.8. In yet another embodiment, the weakly basic compound has a pKa of less than 14, and includes at least one basic nitrogen atom. In yet another embodiment, the weakly basic compound as a pKa of less than 14, a solubility of not more than 1 mg/mL at pH 6.8, and includes a least one basic nitrogen atom. Non-limiting examples of classes of suitable active pharmaceutical ingredients include, but are not limited to analgesics, antihypertensives, antianxiety agents, anticlotting agents, anticonvulsants, anti-diabetic agents, blood glucose-lowering agents, decongestants, antihistamines, anti-inflammatory agents, antitussives, antineoplastics, beta blockers, antirheumatic agents, anti-inflammatories, antipsychotic agents, cognitive enhancers, anti-atherosclerotic agents, anti-obesity agents, anti-impotence agents, anti-infective agents, anti-infective agents, hypnotic agents, anti-Parkinsonism agents, anti-Alzheimer's disease agents, anti-depressants, and antiviral agents, glycogen phosphorylase inhibitors, cholesterol ester transfer protein inhibitors, CNS (central nervous system) stimulants, dopamine receptor agonists, anti-emetics, gastrointestinal agents, psychotherapeutic agents, opioid agonists, opioid antagonists, anti-epileptic drugs, histamine H₂ antagonists, anti-asthmatic agents, smooth muscle relaxants, and skeletal muscle relaxants. Specific examples of analgesics include rofecoxib, celecoxib, morphine, codeine, oxycodone, hydrocodone, diamorphine, pethidine, tramadol, buprenorphene; antihypertensives include prazosin, nifedipine, lercanidipine, amlodipine besylate, trimazosin and doxazosin; specific examples of antianxiety agents include hydroxyzine hydrochloride, lorazepam, buspirone hydrochloride, pazepam, chlordiazepoxide, meprobamate, oxazepam, trifluoperazine hydrochloride, clorazepate dipotassium, diazepam; specific examples of anticlotting agents include abciximab, eptifibatide, tirofiban, lamifiban, clopidogrel, ticlopidine, dicumarol, heparin, and warfarin; specific examples of anticonvulsants include phenobarbital, methylphenobarbital, clobazam, clonazepam, clorezepate, diazepam, midazolam, lorazepam, felbamate, carbamezepine, oxcarbezepine, vigabatrin, progabide, tiagabine, topiramate, gabapentin, pregabaln, ethotoin, phenytoin, mephenytoin, fosphenytoin, paramethadione, trimethadione, ethadione, beclamide, primidone, brivaracetam, levetiracetam, seletracetam, ethosuximide, phensuximide, mesuximide, acetazolamide, sulthiame. methazol amide, zonisamide, lamotrigine, pheneturide, phenacemide, valpromide, and valnoctamide; specific examples of antidiabetic agents include repaglinide, nateglinide, metformin, phenformin, rosiglitazone, pioglitazone, troglitazone, miglitol, acarbose, exanatide, vildagliptin, and sitagliptin; specific examples of blood glucose-lowering agent include tolbutamide, acetohexamide, tolazamide, glyburide, glimepiride, gliclazide, glipizide and chlorpropamide; specific examples of decongestants include pseudoephedrine, phenylephrine, and oxymetazoline; specific examples of antihistamines include mepyramine, antazoline, diphenhydramine, carbinoxamine, doxylamine, clemastine, dimenhydrinate, pheniraminc, chloφheniramine, dexchlorpheniramine, brompheniramine, tripolidine, cyclizine, chlorcyclizine, hydroxyzine, meclizine, promethazine, trimeprazine, cyproheptadine, azatadine, and ketotifen; specific examples of antitussives include dextromethorphan, noscapine, ethyl morphine, and codeine; specific examples of antineoplastics include chlorambucil, lomustine, tubulazole and echinomycin; specific examples of anti-inflammatory agents include betamethasone, prednisolone, aspirin, piroxicam, valdecoxib, carprofen, celecoxib, flurbiprofen and (+)-N-{4-[3-(4-fluorophenoxy)phenoxy]-2-cyclopenten-l -yl}-N-hyroxyurca; specific examples of beta-blockers include timolol and nadolol: specific examples of antitussives include dextromethorphan, noscapine, ethyl morphine, theobromine, and codeine; specific examples of anti-neoplasties include actinomycin, dactinomycin, doxorubicin, daunorubicin, epirurubicin, bleomycin, plicamycin, and mitomycin; specific examples of beta-blockers include alprenolol, carteolol, levobunolol, mepindolol, metipranolol, nadolol, oxprenolol, penbutolol, pindolol, propranolol, sotalol, timolol, acebutolol, atenolol, betaxolol, bisoprolol, esmolol, metoprolol, nebivolol, carvedilol, celiprolol, labetalol, and butaxemine; specific examples of antirheumatic agents include adalimumab, azathioprine, chloroquine, hydroxychloroquine, cyclosporine, D-penicillamine, etanercept, sodium aurothiomalate, auranofin, infliximab, leflunomide, methotrexate, minocycline, sulfasalazine; specific examples of anti -inflammatories include steroidal and nonsteroidal anti-inflammatory drugs such as hydrocortisone, prednisone, prednisolone, methylprednisolone, dexamethasone, betamethasone, triamcinolone, beclomethasonc, aldosterone, acetaminophen, amoxiprin, benorilate, diflunisal, faislamine, diclofenac, aceclofcnac. acemetacin, bromfenac, etodolac, indomethacin, nabumctonc, sulindac, tolmetin, carprofen, ketorolac, mefenamic acid, phenylbutazone, azaanti-inflammatoricspropazone, matamizole, oxyphenbutazone, sulfinprazone, piroxicam, lornoxicam, meloxicam, tcnoxicam, celecoxib, etoricoxib, lumiricoxib, parecoxib, rofecoxib, valdecoxib, and numesulide; specific examples of antipsychotic agents include iloperidone, ziprasidone, olanzepine, thiothixene hydrochloride, fluspirilene, risperidone and penfluridole; a specific example of a cognitive enhancer includes ampakine; specific examples of anti-atherosclerotic, cardiovascular and/or cholesterol reducing agents include atorvastatin calcium, cerivastatin, fluvastatin, lovastatin, mevastatin, pitavastatin, pravastatin, rosuvastatin, and simvastatin; specific examples of antiobesity agents include dexadrine, dexfenfluramine, fenfluramine, phentermine, orlistat, acarbose, and rimonabant; specific examples of anti-impotence agents include sildenafil and sildenafil citrate; specific examples of anti-infective agents such as antibacterial, antiviral, antiprotozoal, antihelminthic and antifungal agents include carbenicillin indanyl sodium, bacampicillin hydrochloride, troleandomycin, doxycycline hyclate, ampicillin, penicillin G, azithromycin, oxytetracycline, minocycline, erythromycin, clarithromycin, spiramycin, acyclovir, nelfinavir, virazole, benzalkonium chloride, chlorhexidine, econazole, terconazole, fluconazole, voriconazole, griseofulvin, metronidazole, thiabendazole, oxfendazole, morantel, cotrimoxazole; specific examples of hypnotic agents include alfaxalone and etomidate; specific examples of anti-Parkinsonism agents include levodopa, bromocriptine, pramipexole, ropinirole, pergolide, and selegiline; anticholinergics such as trihexyphenidyl, benztropine mesylate, procyclidine, biperiden, andethopropazine; antihistamines such as diphenhydramine and dorphenadrine; and amantadine; specific examples of anti-Alzheimer's disease agents include donepezil rivastigmine, galantamine, tacrine; specific examples of antibiotics include minocycline, rifampin, erythromycin, nafcillin, cefazolin, imipenem, aztreonam, gentamicin, sulfamethoxazole, vancomycin, ciprofloxacin, trimethoprim, metronidazole, clindamycin, telcoplanin, mupirocin, azithromycin, clarithromycin, ofloxacin, lomefloxacin, norfloxacin, nalidixic acid, sparfloxacin, pefloxacin, amifloxacin, enoxacin, fleroxacin, ternafloxacin, tosufloxacin, clinafloxacin, sulbactam, clavulanic acid, amphotericin B, fluconazole, itraconazole, ketoconazole, nystatin; specific examples of anti-depressants include isocarboxazid; phenelzine; tranylcypromine; specific examples of antiviral agents include azidovudine (AZT), didanosine (dideoxyinosine, ddl), d4T, zalcitabine (dideoxycytosine, ddC), nevirapine, lamivudine (epivir, 3TC), saquinavir (Invirase), ritonavir (Norvir). indinavir (Crixivan), delavirdine (Rescriptor); specific examples of glycogen phosphorylase inhibitors include [R-(R*S*)]-5-chloro-N-[2-hydroxy-3- {methoxymethylamino}-3-oxo-l - (phenylmethyl)propyl-l H-indole-2-carboxamide and 5-chloro- 1 H-indole-2-carboxylic acid [(l S)-benzyl-(2R)-hydroxy-3-((3R,4S)-dihydroxy-pyrrolidin-l-yl-)-3-o- xypropyl] amide; specific examples of cholesterol ester transfer protein inhibitors include [2R,4S] 4-[(3,5-bis-trifluoromethyl-benzyl)-methoxycarbonyl-amino]-2-ethyl-6-trifluoromethyl-3,4-dihydro-2H-quinoline-1-carboxylic acid ethyl ester, [2R,4S] 4-[acetyl-(3,5-bis-trifluoromethyl-benzyl)-amino]-2-ethyl-6-trifluoromethyl-3,4-dihydro-2H-quinoline- 1 -carboxylic acid isopropyl ester, [2R, 4S] 4-[(3,5-Bis-trifluoromethyl-benzyl)-methoxycarbonyl-amino]-2-eth-yl-6-trifluoromethyl-3,4-dihydro-2H-quinoline-l -carboxylic acid isopropyl ester; specific examples of CNS stimulants include caffeine and methylphenidate; specific examples of dopamine receptor agonists include cabergoline and pramipexole; specific examples of antiemetics include dolasetron, granisetron, ondansetron, tropisetron, palonosetron, domperidone, droperidol, dimenhydrinate, haloperidol, chlorpromezine, promethazine, prochlorperizine, metoclopramide, and alizapride; specific examples of gastrointestinal agents include loperamide and cisapride; specific examples of psychotherapeutic agents include chlorpromazine, thioridazine, prochlorperizine, haloperidol, alprazolam, amitriptyline, bupropion, buspirone, chlordiazepoxide, citalopram, clozapine, diazepam, fluoxetine, fluphenazine, fluvoxamine, hydroxyzine, lorezapam, loxapine, mirtazepine, molindone, nefazodone, nortriptyline, olanzepine, paroxetine, phenelzine, quetiapine, risperidone, sertraline, thiothixene, tranylcypromine, trazodone, venlafaxine, and ziprasidone; specific examples of opioid agonists include hydromorphone, fentanyl, methadone, morphine, oxycodone, and oxymorphone; specific examples of opioid antagonists include naltrexone; specific examples of anti-epileptic drugs include sodium valproate, nitrazepam, phenytoin; specific examples of histamine H₂ antagonists include famotidine, nizatidine, cimetidine, ranitidine; specific examples of anti-asthmatic agents include albuterol, montelukast sodium; specific examples of smooth muscle relaxants include nicorandil, iloperidone, and clonazepam; and specific examples of skeletal muscle relaxants include diazepam, lorazepam, baclofen, carisoprodol, chlorzoxazone, cyclobenzaprine, dantrolene, nietaxalone, orphenadrine, pancuronium, tizanidine, dicyclomine, clonidine, and gabapentin. Each named drug should be understood to include the free form of the drug, as well as pharmaceutically acceptable salts, solvates, esters, and prodrugs thereof.

As used herein, the term "weakly acidic compound" as well as reference to any specific new chemical entity, drug, or active pharmaceutical ingredient, includes the acid, pharmaceutically acceptable salts, polymorphs, stereoisomers, solvates, esters and mixtures thereof, which is a chemical base in which deprotonation is incomplete in aqueous medium. In one embodiment, the weakly acidic drug of the compositions of the present invention can refer to a compound having at least one pKa of less than 14, wherein pKa can be measured or by calculation. In another embodiment, the weakly acidic compound of the compositions of the present invention can refer to a compound having at least one pKa of less than 14, which has a pH dependent solubility between physiological pH with a lower solubility at lower pH. In another embodiment, the weakly acidic drug of the compositions of the present invention can refer to a compound having at least one pKa of 0.0-10.0, which has a pH dependent solubility between physiological pH of 1.0-8.0 with a lower solubility around pH 1.0-2.0. In another embodiment, the weakly acid compound has a solubility of not more than about 1 mg/mL at pH 1.0-2.0. In another embodiment, the weakly acidic compound includes at least one acidic functional group. In yet another embodiment, the weakly acidic compound has at least one pKa of less than 14, and a solubility of not more than about 1 mg/mL at pH 1.2. In yet another embodiment, the weakly acidic compound has a pKa of less than 14, and includes at least one acidic functional group. In yet another embodiment, the weakly acidic compound has a pKa of less than 14, a solubility of not more than 1 mg/mL at pH 1.2, and includes a least one acidic functional group. Representative weakly acidic pharmaceutical drugs include but not limited to: acetaminophen, acetaminosalol, acetazolamide, acitretin, acrivastine, ampicillin, arbutin, azelaic acid, benzoyl peroxide, caffeic acid, chlorothiazide, chlorpropamide, ciclopirox, ciprofloxacin, cromolyn, ethacrynic acid, ferulic acid, furosemide, hydroquinone, ibuprofen, kojic acid, methotrexate, penicillamine, penicillins, pentobarbital, phenobarbital, phenytoin, perindopril, propylthiouracil, rabeprazole, retinoic acid, risedronic acid, salicylic acid, sulfacetamide, sulfabenz, sulfabenzamide, sulfabromomethazine. sulfachlorpyridazine, sulfacytine, sulfadimethoxine, sulfadoxine, sulfaguanole, sulfalene, sulfamethizole, sulfamethoxazole, sulfapyrazine, sulfapyridine, sulfasalazine, sulfasomizole, sulfathiazole, theophylline, thioctic acid, 6,8-dimercaptooctanoic acid (dihydrolipoic acid), tolbutamide, triclosan, urocanic acid, ursodiol, and warfarin. Each named drug should be understood to include the free form of the drug, as well as pharmaceutically acceptable salts, solvates, esters, and prodrugs thereof.

The term "neutral or non-ionizable compound" as well as reference to any specific new chemical entity, drug, or active pharmaceutical ingredient, includes polymorphs, stereoisomers, solvates, esters and mixtures thereof. The neutral or non-ionizable API of the compositions of the present invention can refer to a compound that has a neutral form or does not have an ionizable functional group in the pH range of below 14. In one embodiment, the neutral or non-ionizable compound has a pH-independent solubility at pH of -2 to 14.0, In another embodiment, the neutral/non-ionizable compound has a pH-independent solubility at pH of -1 to 12.0. In another embodiment, the neutral/non-ionizable compound has a pH-independent solubility at pH of 0.0 to 10.0. In another embodiment, the neutral/ or non-ionizable compound has a pH-independent solubility at pHs of 1.0 to 8.0. In another embodiment, the neutral or non-ionizable compound has a pH-independent solubility at pH of 1.0 to 8.0 and has a solubility of not more than 1 mg/mL at pH 1.0 to 8.0.

The "water-soluble polymers" included in the present invention refer to polymers that are soluble in aqueous medium with pH range below 14. It may be ionic or neutral polymers with polar or charged functional groups. It does not include insoluble, but swellable polymer such as crosslinked polyacrylic acids (Carbopol^{®}). Water-soluble polymers suitable for use in the present invention include for example, but are not limited thereto: homopolymers and copolymers of N-vinyl lactams, especially homopolymers and copolymers of N-vinyl pyrrolidone, e.g. polyvinylpyrrolidone (PVP), copolymers of N-vinyl pyrrolidone and vinyl acetate or vinyl propionate, lauroyl polyoxylglycerides, polyvinyl caprolactam-polyvinyl acetate-polyethylene glycol graft copolymer marketed such as Soluplus^{®}, polyoxyethylene polyoxypropylene block copolymers or polyoxyethylene polypropyleneglycol (Poloxamer), cellulose esters and cellulose ethers; in particular methylcellulose, hydroxyalkylcelluloses, in particular hydroxypropylcellulose, hydroxyalkylalkylcelluloses, in particular hydroxypropylmethylcellulose, high molecular polyalkylene oxides such as polyethylene oxide and polypropylene oxide and copolymers of ethylene oxide and propylene oxide, poly(hydroxyalkyl acrylates), poly(hydroxyalkyl methacrylates), polyacrylate, polymethylacrylate, polyacrylamides, vinyl acetate polymers such as copolymers of vinyl acetate and crotonic acid, partially hydrolyzed polyvinyl acetate (also referred to as partially saponified "polyvinyl alcohol"), polyvinyl alcohol, oligo- and polysaccharides such as carrageenans, galactomannans and xanthan gum, or mixtures of one or more thereof.

The term "pH sensitive polymer" includes enteric polymers and gastric-soluble polymer defined below.

The term "enteric polymers" included in the present invention have pH dependent solubility in the gastrointestinal tract which have solubility resistance in gastric fluid (at or around pH 1-4) but will have solubility when the pH of the fluid increases such as in the intestinal tract (above pH 5). Examples of enteric polymers useful in the present invention include, but are not limited to, cellulose derivatives such as cellulose acetate phthalate (CAP), hydropropyl methylcellulose phthalate (HPMCP-50 or HPMCP-55), hydroxypropyl methylcellulose acetate succinate (HPMCAS), alkali-soluble acrylic copolymers (Eudragit^{®} L series and Eudragit^{®} S series), polyvinyl acetate phthalate (PVAP), alginates, Carboxymethyl cellulose (CMC) or any combinations thereof.

The term "gastric-soluble polymers" included in the present invention have pH dependent solubility in the gastrointestinal tract which is soluble in gastric fluid (at or around pH 1-4) but will not have solubility when the pH of the fluid increases such as in the intestinal tract (above pH 5). Examples of gastric-soluble polymer enteric polymers useful in the present invention include, but are not limited to, methacrylic acid copolymers (such as Eudragit E^{®}, Eudragit E100^{®}), Eudragit E100 (also referred to as butylmethacylat-(2-dimethylaminoethyl)-methacrylat-methylmethacylat-copolymer (1:2:1), is a copolymer based on (2-dimethylaminoethyl) methacryalate, butyl methacrylate and methyl methacrylate having a mean molecular weight of about 150,000), chitosan and its derivatives (linear polysaccharide composed of randomly distributed β-(1-4)-linked D-glucosamine (deacetylated unit) and N-acetyl-D-glucosamine (acetylated unit), which are made by treating shrimp and other crustacean shells with alkali sodium hydroxide), or other high molecule weight polymer with at least one cationic function group, or any combinations thereof.

The term "pharmaceutically acceptable surfactant" as used herein refers to a pharmaceutically acceptable ionic or non-ionic surfactant. The surfactants included in the present invention have amphiphilic property such that the use will aid in solubilizing the API in solution. The surfactants included in the present invention will increase the wetting and solubilization of an API in a formulation when used together. Examples of surfactants included in the present invention but not limited to are; lauroyl polyoxylglycerides, polyvinyl caprolactam-polyvinyl acetate-polyethylene glycol graft copolymer marketed such as Soluplus^{®}, sodium docusate, polyethylene glycol-26 glycerin marketed as Renex G26^{®}, polyoxyehthylene monostearate, d-α-Tocopheryl polyethylene glycol 1000 succinate (vitamin E TPGS), polyoxyethylene alkyl ethers, e.g. polyoxyethylene lauryl ether, polyoxyethylene cetyl ether, polyoxyethylene stearyl ether, polyoxyethylene stearyl ether; polyoxyethylene alkylaryl ethers, e.g. polyoxyethylene nonylphenyl ether, polyoxyethylene nonylphenyl ether; polyoxyethylene nonylphenyl ether, polyoxyethylene octylphenyl ether; polyethylene glycol fatty acid esters, e.g. PEG-200 monolaurate, PEG-200 dilaurate, PEG-300 dilaurate, PEG-400 dilaurate, PEG-300 distearate, PEG-300 dioleate; alkylene glycol fatty acid mono esters, e.g. propylene glycol monolaurate (Lauroglycol^{®}); sucrose fatty acid esters, e.g. sucrose monostearate, sucrose distearate, sucrose monolaurate, sucrose dilaurate; or sorbitan fatty acid mono esters such as sorbitan mono laurate, sorbitan monooleate, sorbitan monopalmitate, or sorbitan stearate, polyoxyethylene castor oil derivates, e.g. polyoxyethyleneglycerol triricinoleate or polyoxyl 35 castor oil (Cremophor^{®} EL.) or polyoxyethyleneglycerol oxystearate such as polyethylenglycol 40 hydrogenated castor oil (Cremophor^{®} RH 40) or polyethylenglycol 60 hydrogenated castor oil (Cremophor^{®} RH 60); or block copolymers of ethylene oxide and propylene oxide, also known as polyoxyethylene polyoxypropylene block copolymers or polyoxyethylene polypropyleneglycol, such as Poloxamer 124, Poloxamer 188, Poloxamer 237, Poloxamer 388, Poloxamer 407; or a mono fatty acid ester of polyoxyethylene sorbitan, e.g. polyoxyethylene sorbitan monooleate (Tween^{®} 80), polyoxyethylene sorbitan monostearate (Tween^{®} 60), polyoxyethylene sorbitan monopalmitate, polyoxyethylene sorbitan monolaurate (Tween^{®} 20), or mixtures of one or more thereof.

An "aqueous environment" as employed herein generally means the gastrointestinal fluid if in vivo and aqueous test medium if in vitro. More specifically, "aqueous environment" means (1) if the aqueous environment is in vivo and has a pH in the range of 1.0 to 2.0, the stomach; (2) if the aqueous environment is in vivo and has a pH in the range of 6.0 to 8.0, the intestine; A composition according to the invention can be tested in vivo or, more conveniently, tested in vitro as further disclosed and discussed below to ascertain whether it is within the scope of the invention.

"No-sink dissolution in aqueous environment" refer to the total target concentration of compound in the said composition used for dissolution testing in the aqueous environment described above is higher than the solubility of said compound in the aqueous medium.

In one embodiment of the present invention, pharmaceutical solid dispersion compositions comprising water-soluble and enteric polymers combination are formed to provide a relatively pH independent API solubility when used with an poorly water soluble weakly basic API as a dosage form, the composition of water soluble polymer to enteric polymer weight ratio will range from 9.5:0.5 to 0.5: 9.5. Under this embodiment , the solid dispersions of poorly water soluble basic APIs with water-soluble polymer and enteric polymers may be prepared by; co-precipitation technique, direct compression technique, electro spinning technique, extrusion spheronization technique, freeze drying technique, grinding technique, melt extrusion technique, milling technique, solvent evaporation technique, super critical fluid technique and wet granulation technique.

The API(s) that is (are) poorly water soluble included in the pharmaceutical compositions of the present invention will have sufficient amount to be therapeutically effective. The knowledge of therapeutically effective amount for a given API is known to those working in the area related to the art. In the present invention, the API may be present in a weight ratio of API to the combination of water-soluble polymer and enteric polymer in the range of (0.001 :99.99) to (99:1).

The pharmaceutical compositions of the present invention may exist as a dispersion of crystalline API typically of less than 10 µm in diameter, or amorphous API typically of less than 10 µm in diameter in polymer matrix of water-soluble and enteric polymer mixture or as a molecularly dispersed API in polymer matrix of water-soluble and enteric polymer mixture. In the case where the API is in its amorphous form, the amorphous content will be characterized by X-ray diffraction analysis (XRD), Fourier-transform infrared spectroscopy (FT-IR) and differential scanning calorimetry (DSC).

In another embodiment, pharmaceutical solid dispersion compositions comprising water-soluble polymer, enteric polymer and pharmaceutically acceptable surfactant are invented for pH independent API solubility of poorly water soluble weakly basic API. Composition of the excipients may comprise water-soluble polymer and pharmaceutically acceptable surfactant, enteric polymer in the combined (water-soluble polymer and pharmaceutically acceptable surfactant) to enteric polymer weight ratio range of 9.5:0.5 to 0.5: 9.5 and the weight ratio of water-soluble polymer to pharmaceutically acceptable surfactant is in the range from 0.01:1 to 1:0.01.

Under this embodiment, the solid dispersions of poorly water soluble APIs with water-soluble polymer, pharmaceutically acceptable surfactant and enteric polymer may be prepared by; blending technique, co-precipitation technique, direct compression technique, electro spinning technique, extrusion spheronization technique, freeze drying technique, melt extrusion technique, milling technique, solvent evaporation technique and wet granulation technique.

Under this embodiment, the API may be present in a weight ratio of API to the combination of water-soluble polymer, enteric polymer and surfactant/surfactant-like polymer in the range of (0.001:99.99) to (99:1).

In general, a pharmaceutical composition comprising water soluble polymer, enteric and surfactant and a poorly water soluble weakly basic API may be prepared in the following manner, but is not limited to:
1). Mechanical mixing of crystalline API with diameter less than 10 micron with a polymer blend of two or more polymers and/or surfactant prepared as a solid dispersion (e.g. by spray drying, hot melt extruding, lyophilizing etc.)
2). Mechanic mixing of amorphous API with diameter less than 10 micron with a polymer blend of two or more polymers and/or surfactant prepared as a solid dispersion (e.g. by spray drying, hot melt extruding, lyophilizing etc.)
3). Solid dispersion of API, either crystalline or amorphous state, with diameter less than 10 micron within a matrix of two or more polymers and/or surfactant mixture.
4). Solid dispersion of API, either crystalline or amorphous state, with diameter less than 10 micron within a matrix of polymer blend, with surfactant added as external blend.

According to the present invention, pharmaceutical solid dispersion compositions comprising water-soluble and gastric-soluble polymers combination are formed to provide a pH independent API solubility when used with an poorly water soluble weakly acidic API as a dosage form, the composition of hydrophilic polymer to gastric-soluble polymers weight ratio will range from 9.5:0.5 to 0.5:9.5

Under this embodiment, the solid dispersions of poorly water soluble APIs with water-soluble polymer and gastric-soluble polymers may be prepared by; co-precipitation technique, direct compression technique, electro spinning technique, extrusion spheronization technique, freeze drying technique, grinding technique, melt extrusion technique, milling technique, solvent evaporation technique, super critical fluid technique and wet granulation technique.

The API(s) that is (are) poorly water soluble included in the pharmaceutical compositions of the present invention will have sufficient amount to be therapeutically effective. The knowledge of therapeutically effective amount for a given API is known to those working in the area related to the art. In the present invention, the API may be present in a weight ratio of API to the combination of water-soluble polymer and gastric-soluble polymers in the range of (0.001:99.99) to (99:1).

The pharmaceutical composition s of the present invention may exist as a dispersion of crystalline API typically of less than 10 µm in diameter, or amorphous API typically of less than 10 µm in diameter in polymer matrix of water-soluble and gastric-soluble polymers mixture or as a molecularly dispersed API in polymer matrix of water-soluble and gastric-soluble polymers mixture. In the case where the API is in its amorphous form, the amorphous content will be characterized by X-ray diffraction analysis (XRD), Fourier-transform infrared spectroscopy (FT-IR) and differential scanning calorimetry (DSC).

In another embodiment, pharmaceutical compositions comprising water-soluble polymer, gastric-soluble polymers and pharmaceutically acceptable surfactant are developed for pH independent API solubility of poorly water soluble weakly acidic API. Composition of the excipients may consist of water-soluble polymer, pharmaceutically acceptable surfactant and gastric-soluble polymers in the combined (water-soluble polymer and pharmaceutically acceptable surfactant) to gastric-soluble polymers weight ratio range of 9.5:0.5 to 0.5:9.5 and the weight ratio of water-soluble polymer to pharmaceutically acceptable surfactant is in the range from 0.01:1 to 1:0.01.

Under this embodiment, the solid dispersions of poorly water soluble APIs with water-soluble polymer, pharmaceutical acceptable surfactant and gastric-soluble polymers may be prepared by; blending technique, co-precipitation technique, direct compression technique, electro spinning technique, extrusion spheronization technique, freeze drying technique, melt extrusion technique, milling technique, solvent evaporation technique and wet granulation technique.

Under this embodiment, the API may be present in a weight ratio of API to the combination of water-soluble polymer, gastric-soluble polymer and surfactant/surfactant-like polymer in the range of (0.001:99.99) to (99:1).

For this invention, the pharmaceutical compositions consisting of hydrophilic, gastric-soluble polymers and surfactant and a poorly water soluble weakly acidic API may be prepared in the following manner, but is not limited to:
1). Mechanic mixing of crystalline API with diameter less than 10 micron with a polymer blend of two or more polymers and/or surfactant prepared as a solid dispersion (e.g. by spray drying, hot melt extruding, lyophilizing etc.)
2). Mechanic mixing of amorphous API with diameter less than 10 micron with a polymer blend of two or more polymers and/or surfactant prepared as a solid dispersion (e.g. by spray drying, hot melt extruding, lyophilizing etc.)
3). Solid dispersion of API, either crystalline or amorphous state, with diameter less than 10 micron within a matrix of polymer and/or surfactant mixture, or
4). Solid dispersion of API, either crystalline or amorphous state, with diameter less than 10 micron within a matrix of polymer blend, with surfactant added as external blend.

In one embodiment in the present invention, pharmaceutical solid dispersion compositions comprising water-soluble and pH sensitive polymer combination are formed to provide a pH independent API solubility when used with a poorly water soluble neutral / non-ionizable API as a dosage form, the composition of water-soluble polymer to pH sensitive polymer weight ratio will range from 9.5:0.5 to 9.5: 0.5.

Under this embodiment, the solid dispersions of poorly water soluble APIs with water-soluble polymer and pH sensitive polymer may be prepared by; co-precipitation technique, direct compression technique, electro spinning technique, extrusion spheronization technique, freeze drying technique, grinding technique, melt extrusion technique, milling technique, solvent evaporation technique, super critical fluid technique and wet granulation technique.

The API(s) that is (are) poorly water soluble included in the pharmaceutical compositions of the present invention will have sufficient amount to be therapeutically effective. The knowledge of therapeutically effective amount for a given API of such should be known to those working in the area related to the art. In the present invention, the API may be present in a weight ratio of API to the combination of water-soluble polymer and pH sensitive polymer in the range of (0.01:99.99) to (99:1).

The pharmaceutical composition s of the present invention may exist as a dispersion of crystalline API typically of less than 10 µm in diameter, or amorphous API typically of less than 10 µm in diameter in polymer matrix of water-soluble and pH sensitive polymers mixture or as a molecularly dispersed API in polymer matrix of water-soluble and pH sensitive polymer mixture. In the case where the API is in its amorphous form, the amorphous content will be characterized by X-ray diffraction analysis (XRD), Fourier-transform infrared spectroscopy (FT-IR) and differential scanning calorimetry (DSC).

In another embodiment, pharmaceutical compositions comprising water-soluble polymer, pH sensitive polymer and pharmaceutically acceptable surfactant are developed for pH independent API dissolution of poorly water soluble API. Composition of the excipients may consist of water-soluble polymer and pharmaceutically acceptable surfactant, pH sensitive polymer in the combined (water-soluble polymer and pharmaceutically acceptable surfactant) to pH sensitive polymer weight ratio range of 9.5:0.5 to 0.5:9.5 and the weight ratio of water-soluble polymer to pharmaceutically acceptable surfactant is in the range from 0.01:1 to 1:0.01.

Under this embodiment, the solid dispersions of poorly water soluble APIs with water-soluble polymer, pharmaceutically acceptable surfactant and pH sensitive polymer may be prepared by; blending technique, co-precipitation technique, direct compression technique, electro spinning technique, extrusion spheronization technique, freeze drying technique, melt extrusion technique, milling technique, solvent evaporation technique and wet granulation technique.

Under this embodiment, the API may be present in a weight ratio of API to the combination of water-soluble polymer, pH sensitive polymer and surfactant/surfactant-like polymer in the range of (0.01:99.99) to (99:1).

For this invention, the pharmaceutical compositions comprising water-soluble, pH sensitive polymer and surfactant and a poorly water soluble API may be prepared in the following manner, but is not limited to:
1). Mechanic mixing of crystalline API with diameter less than 10 micron with a polymer blend of two or more polymers and/or surfactant prepared as a solid dispersion (e.g. by spray drying, hot melt extruding, lyophilizing etc.)
2). Mechanic mixing of amorphous API with diameter less than 10 micron with a polymer blend of two or more polymers and/or surfactant prepared as a solid dispersion (e.g. by spray drying, hot melt extruding, lyophilizing etc.)
3). Solid dispersion of API, either crystalline or amorphous state, with diameter less than 10 micron within a matrix of polymer and/or surfactant mixture, or
4). Solid dispersion of API, either crystalline or amorphous state, with diameter less than 10 micron within a matrix of polymer blend, with surfactant added as external blend.

It is an object to provide a solid dispersion, wherein the polymer(s) and/or surfactant(s) are present in an amount such that at or after 90 minutes time point after dissolution in non-sink aqueous environment, the ratio of the concentration of said dissolved poorly water-soluble basic compound at pH 1.0-2.0 to that at pH 6.0-8.0 is less than 1.5; and alternatively less than 1.25.

It is an object to provide the solid dispersion of anyone of the above points, wherein the pharmaceutical compositions may be prepared in the following manners:
a). Mechanical mixing of said crystalline API with diameter less than 10 micron with a blend of two or more said polymers and/or said surfactant prepared as a solid dispersion (e.g. by spray drying, hot melt extruding, lyophilizing etc.)
b). Mechanic mixing of said amorphous API with diameter less than 10 micron with a blend of two or more said polymers and/or said surfactant prepared as a solid dispersion (e.g. by spray drying, hot melt extruding, lyophilizing etc.)
c). Dispersion of said API, either crystalline or amorphous state, with diameter less than 10 micron within a solid matrix of said two or more polymers and/or said surfactant mixture.
d). Dispersion of said API, either crystalline or amorphous state, with diameter less than 10 micron within a solid matrix of said two or more polymers, with said surfactant added as an external phase.

It is an object to provide a solid dispersion of anyone of the above points, wherein the enteric polymer is selected from, but not limited to the group consisting of cellulose derivatives such as cellulose acetate phthalate (CAP), hydropropyl methylcellulose phthalate (HPMCP-50 or HPMCP-55), hydroxypropyl methylcellulose acetate succinate (HPMCAS), alkali-soluble acrylic copolymers (Eudragit^{®} L series and Eudragit^{®} S series), polyvinyl acetate phthalate (PVAP), alginates, Carboxymethyl cellulose (CMC) and any combinations thereof.

It is an object to provide a solid dispersion of anyone of the above points, wherein the water soluble polymer is selected from, but not limiedt to the group consisting and homopolymers and copolymers of N-vinyl lactams, escpecially homopolymers and copolymers of N-vinyl pyrrolidone, e.g. polyvinylpyrrolidone (PVP), copolymers of N-vinyl pyrrolidone and vinyl acetate or vinyl propionate, polyvinyl caprolactam-polyvinyl acetate-polyethylene glycol graft copolymer marketed such as Soluplus^{®}, block copolymers of ethylene oxide and propylene oxide, also known as polyoxyethylene polyoxypropylene block copolymers or polyoxyethylene polypropyleneglycol, such as Poloxamer, lauroyl polyoxylglycerides cellulose esters and cellulose ethers; in particular methylcellulose, hydroxyalkylcelluloses, in particular hydroxypropylcellulose, hydroxyalkylalkylcelluloses, in particular hydroxypropylmethylcellulose, high molecular polyalkylene oxides such as polyethylene oxide and polypropylene oxide and copolymers of ethylene oxide and propylene oxide, vinyl acetate polymers such as copolymers of vinyl acetate and crotonic acid, partially hydrolyzed polyvinyl acetate (also referred to as partially saponified "polyvinyl alcohol"), polyvinyl alcohol, oligo- and polysaccharides such as carrageenans, galactomannans and xanthan gum, and mixtures of one or more thereof.

It is an object to provide a solid pharmaceutical dispersion of any one of the points above comprising at least one pharmaceutically acceptable surfactant, wherein said pharmaceutically acceptable surfactant is selected from but not limited to the group consisting of polyoxyethylene alkyl ethers, polyoxyethylene alkylaryl ethers, polyethylene glycol fatty acid esters, alkylene glycol fatty acid mono esters, sucrose fatty acid esters, sorbitan fatty acid mono esters lauroyl polyoxylglycerides, polyvinyl caprolactam-polyvinyl acetate-polyethylene glycol graft copolymer marketed such as Soluplus^{®}, sodium docusate, polyethylene glycol-26 glycerin marketed as Renex G26^{®}, polyoxyehthylene monostearate, d-α-Tocopheryl polyethylene glycol 1000 succinate (vitamin E TPGS), polyoxyethylene alkyl ethers, polyethylene glycol fatty acid esters, alkylene glycol fatty acid mono esters, sucrose fatty acid esters, sorbitan fatty acid mono esters, sorbitan stearate, polyoxyethylene castor oil derivatives, or polyoxyethyleneglycerol oxystearate or block copolymers of ethylene oxide and propylene oxide, also known as polyoxyethylene polyoxypropylene block copolymers or polyoxyethylene polypropyleneglycol, such as Poloxamer^{®} or a mono fatty acid ester of polyoxyethylene sorbitan, e.g. polyoxyethylene sorbitan monooleate (Tween^{®} 80), or mixtures of one or more thereof and mixtures of one or more thereof.

It is an object of this disclosure to provide a use of the solid dispersion according to anyone of the points above together with other pharmaceutical acceptable excipients for preparation of a pharmaceutical dosage form for oral administration to a mammal.
1. It is still another object of this disclosure to provide a solid dispersion, which comprises of at least one poorly water-soluble acidic compound (API) with at least one pharmaceutically acceptable water-soluble polymer, at least one gastric-soluble polymer, and/or at least one pharmaceutically acceptable surfactant;
   wherein in the absence of said solid dispersion, the poorly water-soluble acidic compound has at least one pKa (acid) within the range of 0.0-10.0, has a pH-dependent solubility in aqueous environment of pH 1.0-8.0 with the lowest aqueous solubility of <1.0 mg/mL at pH 1.0-2.0;
   wherein, in said solid dispersion, said gastric-soluble polymer(s) is dispersed in said solid dispersion matrix with API, water-soluble polymer(s) and/or surfactant(s) at a solid state;
   wherein, in said solid dispersion, said polymer(s) and/or surfactant(s) are present in an amount such that the concentration of said dissolved poorly water-soluble acidic compound at both pH 1.0-2.0 and pH 6.0-8.0 at or after 90 minutes time point during non-sink dissolution test in aqueous environment is at least 2 fold of the solubility of said API alone in aqueous environment at pH 1.0-2.0 not containing said solid dispersion; and wherein, in said solid dispersion, said polymer(s) and/or surfactant(s) are present in an amount such that at or after 90 minutes time point after non-sink dissolution test in aqueous environment, the ratio of the concentration of said dissolved poorly water-soluble acidic compound at pH 6.0-8.0 to that at pH 1.0-2.0 is less than 2.0.

It is an object to provide the above solid dispersion, wherein drug loading of said poorly water-soluble compound is in the range from 0-95% w/w.

It is an object to provide the above solid dispersion, wherein the weight ratio of said water soluble polymer(s) and/or pharmaceutical acceptable surfactant(s) combination to said gastric-soluble polymer(s) is in the range from 0.5:9.5 to 9.5:0.5; alternatively in the range from 4:1 to 9:1, alternatively in the range from 1:9 to 7:3, and still alternatively in the range from 1:2 to 5:1.

It is an object to provide the above solid dispersion, wherein said weight ratio of surfactant to water soluble polymer is in the range from 0.1:9.9 to 9.9:0.1

It is an object to provide the above solid dispersion, wherein said aqueous environment is a gastric-intestinal fluid.

It is an object to provide the above solid dispersion, wherein the aqueous environment is an in-vitro test medium.

It is an object to provide the above solid dispersion, wherein the polymer(s) and/or surfactant(s) are present in an amount such that at or after 90 minutes time point after dissolution in non-sink aqueous environment, the ratio of the concentration of said dissolved poorly water-soluble acidic compound at pH 6.0-8.0 to that at pH 1.0-2.0 is less than 1.5, alternatively less than 2.5.

It is an object to provide the above solid dispersion, wherein non-sink dissolution test of said solid dispersion in aqueous environment uses a total API target concentration higher than said solubility of API alone in aqueous environment at pH 1.0-2.0.

2. It is yet an object to provide the above solid dispersion, which comprises of at least one poorly water-soluble acidic compound (API) with at least one pharmaceutically acceptable water-soluble polymer, and at least one gastric-soluble polymer,
wherein, in the absence of said solid dispersion, the poorly water-soluble acidic compound has at least one pKa (acid) within the range of 0.0-10.0, has a pH-dependent solubility in aqueous environment of pH 1.0-8.0 with the lowest aqueous solubility of <1.0 mg/mL at pH 1.0-2.0,
wherein, in said solid dispersion, said gastric-soluble polymer(s) is dispersed in said solid dispersion matrix with API, water-soluble polymer(s),
wherein, in said solid dispersion, said polymer(s) are present in an amount such that the concentration of said dissolved poorly water-soluble acidic compound at both pH 1.0-2.0 and pH 6.0-8.0 at or after 90 minutes time point during non-sink dissolution test in aqueous environment is at least 2 fold of the solubility of said API alone in aqueous environment at pH 1.0-2.0 not containing said solid dispersion,
wherein, in said solid dispersion, said polymer(s) are present in an amount such that at or after 90 minutes time point after non-sink dissolution test in aqueous environment, the ratio of the concentration of said dissolved poorly water-soluble acidic compound at pH 6.0-8.0 to that at pH 1.0-2.0 is less than 2.0.

It is an object to provide the above solid dispersion, wherein the drug loading of said poorly water-soluble compound is in the range from 0-95% w/w.

It is an object to provide the above solid dispersion, wherein the weight ratio of said water soluble polymer(s) to said gastric-soluble polymer(s) is in the range from 0.5:9.5 to 9.5:0.5, alternatively in the range from 4:1 to 9:1, alternatively in the e range from 1:9 to 7:3, and still alternatively in the range from 1:2 to 5:1.

It is an object to provide the above solid dispersion wherein the aqueous environment is a gastric-intestinal fluid.

It is an object to provide the above solid dispersion wherein the aqueous environment is in-vitro test medium.

It is an object to provide the above solid dispersion, wherein the polymer(s) are present in an amount such that at or after 90 minutes time point after dissolution in non-sink aqueous environment, the ratio of the concentration of said dissolved poorly water-soluble acidic compound at pH 6.0-8.0 to that at pH 1.0-2.0 is less than 1.5, alternatively less than 1.25.

It is an object to provide the above solid dispersion, wherein said non-sink dissolution test of said solid dispersion in aqueous environment uses a total API target concentration higher than said solubility of API alone in aqueous environment at pH 1.0-2.0.

3. It is yet an object to provide the above solid dispersion, which comprises of at least one poorly water-soluble acidic compound (API) with at least one pharmaceutically acceptable water-soluble polymer, at least one gastric-soluble polymer, and at least one pharmaceutically acceptable surfactant,
wherein, in the absence of said solid dispersion, the poorly water-soluble acidic compound has at least one pKa (acid) within the range of 0.0-10.0, has a pH-dependent solubility in aqueous environment of pH 1.0-8.0 with the lowest aqueous solubility of <1.0 mg/mL at pH 1.0-2.0,
wherein, in said solid dispersion, said enteric polymer(s) is dispersed in said solid dispersion matrix with API, water-soluble polymer(s) and surfactant(s) at a solid state,
wherein, in said solid dispersion, said polymer(s) and surfactant(s) are present in an amount such that the concentration of said dissolved poorly water-soluble acidic compound at both pH 1.0-2.0 and pH 6.0-8.0 at or after 90 minutes time point during non-sink dissolution test in aqueous environment is at least 2 fold of the solubility of said API alone in aqueous environment at pH 1.0-2.0 not containing said solid dispersion. wherein, in said solid dispersion, said polymer(s) and surfactant(s) are present in an amount such that at or after 90 minutes time point after non-sink dissolution test in aqueous environment, the ratio of the concentration of said dissolved poorly water-soluble acidic compound at pH 6.0-8.0 to that at pH 1.0-2.0 is less than 2.0.

It is an object to provide the above solid dispersion, wherein the drug loading of said poorly water-soluble compound is in the range from 0-95% w/w.

It is an object to provide the above solid dispersion , wherein the weight ratio of said water soluble polymer(s) and pharmaceutical acceptable surfactant(s) combination to said gastric-soluble polymer(s) is in the range from 0.5:9.5 to 9.5:0.5, alternatively in the range from 4:1 to 9:1, alternatively in the range from 1:9 to 7:3, alternatively in the range from 1:2 to 5:1, and still alternatively in the range from 0.5:9.5 to 9.9:0.1.

It is an object to provide the above solid dispersion, wherein said aqueous environment is a gastric-intestinal fluid.

It is an object to provide the above solid dispersion, wherein said aqueous environment is an in-vitro test medium.

It is an object to provide the above solid dispersion, wherein, in said composition, said polymer(s) and surfactant(s) are present in an amount such that at or after 90 minutes time point after dissolution in non-sink aqueous environment, the ratio of the concentration of said dissolved poorly water-soluble acidic compound at pH 6.0-8.0 to that at pH 1.0-2.0 is less than 1.5, alternatively less than 1.25.

It is an object to provide the above solid dispersion, wherein said non-sink dissolution test of said solid dispersion in aqueous environment uses a total API target concentration higher than said solubility of API alone in aqueous environment at pH 1.0-2.0.

It is an object to provide the above solid dispersion, wherein said pharmaceutical compositions may be prepared in the following manners:
a). Mechanical mixing of said crystalline API with diameter less than 10 micron with a blend of two or more said polymers and/or said surfactant prepared as a solid dispersion (e.g. by spray drying, hot melt extruding, lyophilizing etc.)
b). Mechanic mixing of said amorphous API with diameter less than 10 micron with a blend of two or more said polymers and/or said surfactant prepared as a solid dispersion (e.g. by spray drying, hot melt extruding, lyophilizing etc.)
c). Dispersion of said API, either crystalline or amorphous state, with diameter less than 10 micron within a solid matrix of said two or more polymers and/or said surfactant mixture, or
d). Dispersion of said API, either crystalline or amorphous state, with diameter less than 10 micron within a solid matrix of said two or more polymers, with said surfactant added as an external phase.

It is an object to provide the above solid dispersion, wherein the said gastric-soluble is selected from the group consisting of methacrylic acid copolymers (such as Eudragit E^{®}, Eudragit E100^{®}), Eudragit E100 (also referred to as butylmethacylat-(2-dimethylaminoethyl)-methacrylat-methylmethacylat-copolymer (1:2:1), is a copolymer based on (2-dimethylaminoethyl) methacryalate, butyl methacrylate and methyl methacrylate having a mean molecular weight of about 150,000), chitosan and its derivatives (linear polysaccharide composed of randomly distributed β-(1-4)-linked D-glucosamine (deacetylated unit) and N-acetyl-D-glucosamine (acetylated unit)), or other high molecule weigh polymer with cationic function group, or any combinations thereof. It is an object to provide the above solid dispersion, wherein said water soluble polymer is selected from the group consisting homopolymers and copolymers of N-vinyl lactams, especially homopolymers and copolymers of N-vinyl pyrrolidone, e.g. polyvinylpyrrolidone (PVP), copolymers of N-vinyl pyrrolidone and vinyl acetate or vinyl propionate, polyvinyl caprolactam-polyvinyl acetate-polyethylene glycol graft copolymer marketed such as Soluplus^{®}, block copolymers of ethylene oxide and propylene oxide, also known as polyoxyethylene polyoxypropylene block copolymers or polyoxyethylene polypropyleneglycol, such as Poloxamer, lauroyl polyoxylglycerides cellulose esters and cellulose ethers; in particular methylcellulose, hydroxyalkylcelluloses, in particular hydroxypropylcellulose, hydroxyalkylalkylcelluloses, in particular hydroxypropylmethylcellulose, high molecular polyalkylene oxides such as polyethylene oxide and polypropylene oxide and copolymers of ethylene oxide and propylene oxide, vinyl acetate polymers such as copolymers of vinyl acetate and crotonic acid, partially hydrolyzed polyvinyl acetate (also referred to as partially saponified "polyvinyl alcohol"), polyvinyl alcohol, oligo- and polysaccharides such as carrageenans, galactomannans and xanthan gum, and mixtures of one or more thereof.

It is an object to provide the above solid dispersion comprising at least one pharmaceutically acceptable surfactant. Wherein said pharmaceutically acceptable surfactant is selected from the group consisting of polyoxyethylene alkyl ethers, polyoxyethylene alkylaryl ethers, polyethylene glycol fatty acid esters, alkylene glycol fatty acid mono esters, sucrose fatty acid esters, sorbitan fatty acid mono esters lauroyl polyoxylglycerides, polyvinyl caprolactam-polyvinyl acetate-polyethylene glycol graft copolymer marketed such as Soluplus^{®}, sodium docusate, polyethylene glycol-26 glycerin marketed as Renex G26^{®}, polyoxyehthylene monostearate, d-α-Tocopheryl polyethylene glycol 1000 succinate (vitamin E TPGS), polyoxyethylene alkyl ethers, polyethylene glycol fatty acid esters, alkylene glycol fatty acid mono esters, sucrose fatty acid esters, sorbitan fatty acid mono esters, sorbitan stearate, polyoxyethylene castor oil derivatives, or polyoxyethyleneglycerol oxystearate or block copolymers of ethylene oxide and propylene oxide, also known as polyoxyethylene polyoxypropylene block copolymers or polyoxyethylene polypropyleneglycol, such as Poloxamer or a mono fatty acid ester of polyoxyethylene sorbitan, e.g. polyoxyethylene sorbitan monooleate (Tween^{®} 80), and mixtures of one or more thereof.

It is an object to provide a use of the above solid dispersion together with other pharmaceutical acceptable excipients for preparation of a pharmaceutical dosage form for oral administration to a mammal.

4. It is yet another object of this disclosure to provide a solid dispersion, which comprises of at least one poorly water-soluble neutral or non-ionizable compound (API) with at least one pharmaceutically acceptable water-soluble polymer, at least one pH-sensitive polymer, and/or at least one pharmaceutically acceptable surfactant.
wherein, in the absence of said solid dispersion, the poorly water-soluble neutral or non-ionizable compound has none detectable (or calculated) pKa within the range of - 1.0 -12.0, and has a pH-independent solubility in aqueous environment of pH 1.0-8.0 with the lowest aqueous solubility of <1.0 mg/mL.
wherein, in said solid dispersion, said pH-sensitive polymer(s) is dispersed in said solid dispersion matrix with API, water-soluble polymer(s) and/or surfactant(s) at a solid state.
wherein, in said solid dispersion, said polymer(s) and/or surfactant(s) are present in an amount such that the concentration of said dissolved poorly water-soluble compound at both pH 1.0-2.0 and pH 6.0-8.0 at or after 90 minutes time point during non-sink dissolution test in aqueous environment is at least 2 fold of the solubility of said API alone in aqueous environment at pH 1.0-8.0 not containing said solid dispersion. wherein, in said solid dispersion, said polymer(s) and/or surfactant(s) are present in an amount such that at or after 90 minutes time point after non-sink dissolution test in aqueous environment, the ratio of the concentration of said dissolved poorly water-soluble compound at pH 6.0-8.0 to that at pH 1.0-2.0 is between 0.5-2.0.

It is an object to provide the above solid dispersion, wherein drug loading of said poorly water-soluble compound is in the range from 0-95% w/w.

It is an object to provide the above solid dispersion, wherein the weight ratio of said water soluble polymer(s) and/or pharmaceutical acceptable surfactant(s) combination to said pH-sensitive polymer(s) is in the range from 0.5:9.5 to 9.5:0.5, alternatively in the range from 1:9 to 7:3, alternatively in the range from 4:1 to 9:1, still alternatively in the range from 1:2 to 5:1.

It is an object to provide the above solid dispersion, wherein said weight ratio of surfactant to water soluble polymer is in the range from 0.1:9.9 to 9.9:0.1.

It is an object to provide the above solid dispersion, wherein said aqueous environment is a gastric-intestinal fluid.

It is an object to provide the above solid dispersion, wherein said aqueous environment is an in-vitro test medium.

It is an object to provide the above solid dispersion, wherein the polymer(s) and/or surfactant(s) are present in an amount such that at or after 90 minutes time point after dissolution in non-sink aqueous environment, the ratio of the concentration of said dissolved poorly water-soluble compound at pH 6.0-8.0 to that at pH 1.0-2.0 is between 0.6-1.5, and alternatively between 0.8-1.25.

It is an object to provide the above solid dispersion, wherein said non-sink dissolution test of said solid dispersion in aqueous environment uses a total API target concentration higher than said solubility of API alone in aqueous environment at pH 1.0-8.0.

5. It is yet an object to provide the above solid dispersion, which comprises of at least one poorly water-soluble neutral or non-ionizable compound (API) with at least one pharmaceutically acceptable water-soluble polymer, and at least one pH-sensitive polymer,
wherein, in the absence of said solid dispersion, the poorly water-soluble neutral or non-ionizable compound has none detectable (or calculated) pKa within the range of - 1.0 -12.0, and has a pH-independent solubility in aqueous environment of pH 1.0-8.0 with the lowest aqueous solubility of <1.0 mg/mL,
wherein, in said solid dispersion, said pH-sensitive polymer(s) is dispersed in said solid dispersion matrix with API, water-soluble polymer(s) at a solid state,
wherein, in said solid dispersion, said polymer(s) and/or surfactant(s) are present in an amount such that the concentration of said dissolved poorly water-soluble compound at both pH 1.0-2.0 and pH 6.0-8.0 at or after 90 minutes time point during non-sink dissolution test in aqueous environment is at least 2 fold of the solubility of said API alone in aqueous environment at pH 1.0-8.0 not containing said solid dispersion, wherein, in said solid dispersion, said polymer(s) and/or surfactant(s) are present in an amount such that at or after 90 minutes time point after non-sink dissolution test in aqueous environment, the ratio of the concentration of said dissolved poorly water-soluble compound at pH 6.0-8.0 to that at pH 1.0-2.0 is between 0.5-2.0.

It is an object to provide the above solid dispersion, wherein the drug loading of said poorly water-soluble compound is in the range from 0-95% w/w.

It is an object to provide the above solid dispersion wherein the weight ratio of said water soluble polymer(s) to said pH-sensitive polymer(s) is in the range from 0.5:9.5 to 9.5:0.5, alternatively in the range from 1:9 to 7:3, alternatively in the range from 4:1 to 9:1, and still alternatively in the range from 1:2 to 5:1.

It is an object to provide the above solid dispersion, wherein said aqueous environment is a gastric-intestinal fluid.

It is an object to provide the above solid dispersion, wherein said aqueous environment is an in-vitro test medium

It is an object to provide the above solid dispersion, wherein, said polymer(s) are present in an amount such that at or after 90 minutes time point after dissolution in non-sink aqueous environment, the ratio of the concentration of said dissolved poorly water-soluble compound at pH 6.0-8.0 to that at pH 1.0-2.0 is between 0.6-1.5, alternatively between 0.8-1.25.

It is an object to provide the above solid dispersion, wherein said non-sink dissolution test of said solid dispersion in aqueous environment uses a total API target concentration higher than said solubility of API alone in aqueous environment at pH 1.0-8.0.

6. It is yet an object to provide the above solid dispersion, which comprises of at least one poorly water-soluble neutral or non-ionizable compound (API) with at least one pharmaceutically acceptable water-soluble polymer, at least one pH-sensitive polymer, and at least one pharmaceutically acceptable surfactant,
wherein, in the absence of said solid dispersion, the poorly water-soluble neutral or non-ionizable compound has none detectable (or calculated) pKa within the range of -1.0 -12.0, and has a pH-independent solubility in aqueous environment of pH 1.0-8.0 with the lowest aqueous solubility of <1.0 mg/m,
wherein, in said solid dispersion, said pH-sensitive polymer(s) is dispersed in said solid dispersion matrix with API, water-soluble polymer(s) and surfactant(s) at a solid state,
wherein, in said solid dispersion, said polymer(s) and surfactant(s) are present in an amount such that the concentration of said dissolved poorly water-soluble compound at both pH 1.0-2.0 and pH 6.0-8.0 at or after 90 minutes time point during non-sink dissolution test in aqueous environment is at least 2 fold of the solubility of said API alone in aqueous environment at pH 1.0-8.0 not containing said solid dispersion, wherein, in said solid dispersion, said polymer(s) and surfactant(s) are present in an amount such that at or after 90 minutes time point after non-sink dissolution test in aqueous environment, the ratio of the concentration of said dissolved poorly water-soluble compound at pH 6.0-8.0 to that at pH 1.0-2.0 is between 0.5- 2.0.

It is an object to provide the above solid dispersion, wherein the drug loading of said poorly water-soluble compound is in the range from 0-95% w/w.

It is an object to provide the above solid dispersion, wherein the weight ratio of said water soluble polymer(s) and pharmaceutical acceptable surfactant(s) combination to said pH-sensitive polymer(s) is in the range from 0.5:9.5 to 9.5:0.5, alternatively in the range from 1:9 to 7:3, alternatively in the range from 4:1 to 9:1, alternatively in the range from 1:2 to 5:1, and still alternatively in the range from 0.5:9.5 to 9.9:0.1.

It is an object to provide the above solid dispersion, wherein said aqueous environment is a gastric-intestinal fluid.

It is an object to provide the above solid dispersion, wherein said aqueous environment is an in-vitro test medium.

It is an object to provide the above solid dispersion, wherein said polymer(s) and surfactant(s) are present in an amount such that at or after 90 minutes time point after dissolution in non-sink aqueous environment, the ratio of the concentration of said dissolved poorly water-soluble compound at pH 6.0-8.0 to that at pH 1.0-2.0 is between 0.6-1.5, alternatively between 0.8-1.25.

It is an object to provide the above solid dispersion, wherein said non-sink dissolution test of said solid dispersion in aqueous environment uses a total API target concentration higher than said solubility of API alone in aqueous environment at pH 1.0-8.0.

It is an object to provide the above solid dispersion, wherein said pharmaceutical compositions may be prepared in the following manners:
1). Mechanical mixing of said crystalline API with diameter less than 10 micron with a blend of two or more said polymers and/or said surfactant prepared as a solid dispersion (e.g. by spray drying, hot melt extruding, lyophilizing etc.),
2). Mechanic mixing of said amorphous API with diameter less than 10 micron with a blend of two or more said polymers and/or said surfactant prepared as a solid dispersion (e.g. by spray drying, hot melt extruding, lyophilizing etc.),
3) Dispersion of said API, either crystalline or amorphous state, with diameter less than ten micron within a solid matrix of said two or more polymers and/or said surfactant mixture, or.
4) Dispersion of said API, either crystalline or amorphous state, with diameter less than ten micron within a solid matrix of said two or more polymers, with said surfactant added as an external phase.

It is an object to provide the above solid dispersion, wherein the said pH-sensitive polymer is selected from, but not limited to the group consisting methacrylic acid copolymers (such as Eudragit E^{®}, Eudragit E100^{®}), Eudragit E100 (also referred to as butylmethacylat-(2-dimethylaminoethyl)-methacrylat-methylmethacylat-copolymer (1:2:1), is a copolymer based on (2-dimethylaminoethyl)methacryalate, butyl methacrylate and methyl methacrylate having a mean molecular weight of about 150,000), chitosan and its deritives (linear polysaccharide composed of randomly distributed β-(1-4)-linked D-glucosamine (deacetylated unit) and N-acetyl-D-glucosamine (acetylated unit)), or other high molecule weigh polymer with cationic function group, cellulose derivatives such as cellulose acetate phthalate (CAP), hydropropyl methylcellulose phthalate (HPMCP-50 or HPMCP-55), hydroxypropyl methylcellulose acetate succinate (HPMCAS), alkali-soluble acrylic copolymers (Eudragit^{®} L series and Eudragit^{®} S series), polyvinyl acetate phthalate (PVAP), alginates, Carboxymethyl cellulose (CMC), or mixtures of one or more thereof.

It is an object to provide the above solid dispersion, wherein said water soluble polymer is selected from the group consisting homopolymers and copolymers of N-vinyl lactams, especially homopolymers and copolymers of N-vinyl pyrrolidone, e.g. polyvinylpyrrolidone (PVP), copolymers of N-vinyl pyrrolidone and vinyl acetate or vinyl propionate, polyvinyl caprolactam-polyvinyl acetate-polyethylene glycol graft copolymer marketed such as Soluplus^{®}, block copolymers of ethylene oxide and propylene oxide, also known as polyoxyethylene polyoxypropylene block copolymers or polyoxyethylene polypropyleneglycol, such as Poloxamer, lauroyl polyoxylglycerides cellulose esters and cellulose ethers; in particular methylcellulose, hydroxyalkylcelluloses, in particular hydroxypropylcellulose, hydroxyalkylalkylcelluloses, in particular hydroxypropylmethylcellulose, high molecular polyalkylene oxides such as polyethylene oxide and polypropylene oxide and copolymers of ethylene oxide and propylene oxide, vinyl acetate polymers such as copolymers of vinyl acetate and crotonic acid, partially hydrolyzed polyvinyl acetate (also referred to as partially saponified "polyvinyl alcohol"), polyvinyl alcohol, oligo- and polysaccharides such as carrageenans, galactomannans and xanthan gum, or mixtures of one or more thereof.

It is an object to provide the above solid dispersion , comprising at least one pharmaceutically acceptable surfactant, wherein said pharmaceutically acceptable surfactant is selected from the group consisting of polyoxyethylene alkyl ethers, polyoxyethylene alkylaryl ethers, polyethylene glycol fatty acid esters, alkylene glycol fatty acid mono esters, sucrose fatty acid esters, sorbitan fatty acid mono esters lauroyl polyoxylglycerides, polyvinyl caprolactam-polyvinyl acetate-polyethylene glycol graft copolymer marketed such as Soluplus^{®}, sodium docusate, polyethylene glycol-26 glycerin marketed as Renex G26^{®}, polyoxyehthylene monostearate, d-α-Tocopheryl polyethylene glycol 1000 succinate (vitamin E TPGS), polyoxyethylene alkyl ethers, polyethylene glycol fatty acid esters, alkylene glycol fatty acid mono esters, sucrose fatty acid esters, sorbitan fatty acid mono esters, sorbitan stearate, polyoxyethylene castor oil derivatives, or polyoxyethyleneglycerol oxystearate or block copolymers of ethylene oxide and propylene oxide, also known as polyoxyethylene polyoxypropylene block copolymers or polyoxyethylene polypropyleneglycol, such as Poloxamer or a mono fatty acid ester of polyoxyethylene sorbitan, e.g. polyoxyethylene sorbitan monooleate (Tween^{®} 80), and mixtures of one or more thereof.

It is an object to provide use of the above solid dispersion according together with other pharmaceutical acceptable excipients for preparation of a pharmaceutical dosage form for oral administration to a mammal.

Examples and standard example for comparison in detailed description of the present invention are shown below, however, the invention is not limited to the illustrated description given thereto.

### Example 1(comparative example)

The poorly water soluble, weakly basic API, prasugrel, 627.44 mg, was dissolved in 100 mL of methanol to make up a stock solution with a concentration of around 6.25 mg/mL. From the prepared prasugrel stock solution, 20.8 mL was added to 10 g of 5% w/w of hydroxypropyl methyl cellulose (HPMC 603: supplied by Shin-Etsu Chemical Co. Ltd.) solution in methanol, while stirring in a beaker. The solution was transferred to a petri dish and heated on a hot plate at 70 degree Celsius until the solvent was evaporated and a film was formed. The film was removed and collected in a vial.

### Example 2 (comparative example))

Prasugrel, 625.14 mg, was dissolved in 100 mL of methanol to make up a stock solution of around 6.25 mg/mL. Separately, 12.5 g of Soluplus^{®} (supplied by BASF) was dissolved in 100.14 g of methanol. While stirring 7.5g of the Soluplus^{®} solution in a beaker, 37.5 mL of the prasugrel solution was added. The mixture was transferred to a petri dish and placed on a hot plate to remove the solvent at 70 degree Celsius. The resultant film was removed and collected in a vial.

### Example 3

Prasugrel, 250.42 mg, was dissolved in 25 mL of methanol. In a beaker, 5.336 g of 12.5% w/w hydroxypropyl methyl cellulose acetate succinate (HPMCAS-LF: supplied by Shin-Etsu Chemical Co. Ltd.) in methanol and 2.664 g of 12.5% w/w Soluplus^{®} in methanol were stirred together. Prasugrel solution was added to the polymer solution and stirred. The solution was transferred to a petri dish and was heated on a hot plate at 70 degree Celsius until the solvent had evaporated completely and a film was formed. The film was removed and collected in a vial.

### Example 4

Prasugrel, 156.25 mg/mL, was dissolved in 25 mL of methanol. In a beaker, 3.14 g of 5% w/w HPMC 603 in methanol, 2.50 g of 12.5% w/w HPMCAS-LF in methanol and 1.25 g of 12.5% w/w Soluplus^{®} in methanol were stirred together. Prasugrel solution was added to the polymeric solution and the mixture was transferred to a petri dish and the solvent was heated on a hot plate until the solvent evaporated completely. The film formed on the dish was removed and collected in a vial.

### Example 5 (comparative example)

Prasugrel (625.14 mg) was dissolved in 100 mL of methanol and was transferred to a petri dish and heated on a hot plate at 70 degree Celsius until the solvent evaporated and a film was formed. This is a reference sample as a control.

### Example 6 (comparative example)

The poorly water soluble, weakly basic API, clopidogrel stock solutions (~ 1 mg/mL) and HPMC 603 (~ 2mg/mL) were prepared in reagent alcohol. For the preparation of solid dispersion of clopidogrel-HPMC, in a micro-centrifuge tube, aliquots of clopidogrel and HPMC stocks solutions were pipetted to have 2:8 of clopidogrel: polymer weight ratio, and vortexed. The solvent was removed by placing the microcentrifuge tubes with their lids open in a personal evaporator system, EZ-2 Plus (Genevac, Stone Ridge, NY), set to low boiling point mixture with maximum temperature set to 60°C. When the evaporation was complete, the microcentrifuge tubes were removed from the evaporator and cooled immediately.

### Example 7

Utilizing procedure described in Example 6, clopidogrel-HPMC 603-HPMCAS-LF-Tween 80 film was prepared by applying a solution containing a known concentration of clopidogrel and polymers in reagent alcohol (Approximately 2:4:4:0.5 of clopidogrel: HPMC: HPMCAS: Tween 80 weight ratio) and dry to create a thin film. In this example, film was dried under vacuum by Genevac solvent evaporator.

### Example 8 (comparative example)

Utilizing procedure described in Example 6, clopidogrel-Eudragit EPO film was prepared by applying a solution containing a known concentration of clopidogrel and polymers in reagent alcohol (Approximately 2:8 of clopidogrel: Eudragit EPO weight ratio) and dry to create a thin film. In this example, film was dried under vacuum by Genevac solvent evaporator.

### Example 9 (comparative example)

Utilizing procedure described in Example 6, clopidogrel-Eudragit EPO-Soluplus film was prepared by applying a solution containing a known concentration of clopidogrel and polymers in reagent alcohol (Approximately 2:2:6 of clopidogrel: Eudragit EPO: Solulpus weight ratio) and dry to create a thin film. In this example, film was dried under vacuum by Genevac solvent evaporator.

### Example 10 (comparative example)

Utilizing procedure described in Example 6, a poorly water soluble acidic API, diclofenac-Eudragit E (by Evonik) polymer film was prepared by applying a solution containing a known concentration of diclofenac and Eudragit E polymer in reagent alcohol (Approximately 2:8 of diclofenac: Eudragit E weight ratio) to create a thin film. In this example, film was dried under vacuum by Genevac solvent evaporator.

### Example 11 (comparative example)

Utilizing procedure described in Example 6, diclofenac-PVPVA 64 (Kollidon VA 64 by BASF ) polymer film was prepared by applying a solution containing a known concentration of diclofenac and PVPVA 64 polymer in reagent alcohol (Approximately 2:8 of diclofenac: PVPVA 64 weight ratio) to create a thin film. In this example, film was dried under vacuum by Genevac solvent evaporator.

### Example 12

Utilizing procedure described in Example 6, diclofenac-Eudragit E-PVPVA 64 (Kollidon VA 64 by BASF ) polymer film was prepared by applying solutions containing a known concentration of diclofenac, Eudragit E, and PVPVA 64 polymer in reagent alcohol (Approximately 2:4:4 of diclofenac: Eudragit E: PVPVA 64 weight ratio) and dry to create a thin film. In this example, film was dried under vacuum by Genevac solvent evaporator.

### Example 13 (comparative example)

Utilizing procedure described in Example 6, diclofenac-HPMCAS(LF)-HPMC603 polymer film was prepared by applying solutions containing a known concentration of diclofenac, HPMC, and HPMCAS polymer in reagent alcohol (Approximately 2:4:4 of diclofenac: HPMC: HPMCAS weight ratio) and dry to create a thin film. In this example, film was dried under vacuum by Genevac solvent evaporator.

### Example 14 (comparative example)

Utilizing procedure described in Example 6, a poorly water-soluble acidic API, ibuprofen -Eudragit E (by Evonik) polymer film was prepared by applying a solution containing a known concentration of ibuprofen and Eudragit E polymer in reagent alcohol (Approximately 2:8 of diclofenac: Eudragit E weight ratio) to create a thin film. In this example, film was dried under vacuum by Genevac solvent evaporator.

### Example 15 (comparative example)

Utilizing procedure described in Example 6, ibuprofen- Soluplus^{®} (supplied by BASF ) polymer film was prepared by applying a solution containing a known concentration of ibuprofen and Soluplus^{®} polymer in reagent alcohol (Approximately 2:8 of ibuprofen: Soluplus^{®} weight ratio) to create a thin film. In this example, film was dried under vacuum by Genevac solvent evaporator.

### Example 16

Utilizing procedure described in Example 6, ibuprofen-Eudragit E- Soluplus^{®} polymer film was prepared by applying a solution containing a known concentration of ibuprofen, Eudragit E, and Soluplus^{®} polymer in reagent alcohol (Approximately 2:4:4 of ibuprofen: Eudragit E: Soluplus^{®} weight ratio) to create a thin film. In this example, film was dried under vacuum by Genevac solvent evaporator.

### Example 17

Utilizing procedure described in Example 6, ibuprofen-Eudragit E- Soluplus^{®} -HPMC 603 film was prepared by applying a solution containing a known concentration of ibuprofen, Eudragit E, HPMC and Soluplus^{®} polymer in reagent alcohol (Approximately 2:4:2:2 of ibuprofen: Eudragit E: Soluplus^{®}:HPMC weight ratio) to create a thin film. In this example, film was dried under vacuum by Genevac solvent evaporator.

### Example 18

Utilizing procedure described in Example 6, ibuprofen-Eudragit E- Soluplus^{®} -Span 20 film was prepared by applying a solution containing a known concentration of ibuprofen, Eudragit E, span 20 and Soluplus^{®} polymer in reagent alcohol (Approximately 2:4:4:1 of ibuprofen: Eudragit E: Soluplus^{®}: Span 20 weight ratio) to create a thin film. In this example, film was dried under vacuum by Genevac solvent evaporator.

### Example 19

Utilizing procedure described in Example 6, ibuprofen-Eudragit E- HPMC 603 was prepared by applying a solution containing a known concentration of ibuprofen, Eudragit E, HPMC 603 polymer in reagent alcohol (Approximately 2:2:6 of ibuprofen: Eudragit E: HPMC weight ratio) to create a thin film. In this example, film was dried under vacuum by Genevac solvent evaporator.

### Example 20 (comparative example)

Utilizing procedure described in Example 6, Ibuprofen-HPMCAS(LF)- HPMC 603 was prepared by applying a solution containing a known concentration of ibuprofen, HPMCAS, HPMC 603 polymer in reagent alcohol (Approximately 2:4:4 of ibuprofen: HPMCAS: HPMC weight ratio) to create a thin film. In this example, film was dried under vacuum by Genevac solvent evaporator.

### Example 21 (comparative example)

Utilizing procedure described in Example 6, apixaban-HPMCAS-LF was prepared by applying a solution containing a known concentration of apixaban and HPMCAS-LF polymer in reagent alcohol (Approximately 2:8 of apixaban: HPMCAS-LF weight ratio) to create a thin film. In this example, film was dried under vacuum by Genevac solvent evaporator.

### Example 22(comparative example)

Utilizing procedure described in Example 6, a neural form/non-ionizable API, apixaban-HPMC 603 was prepared by applying a solution containing a known concentration of apixaban and HPMC 603 polymer in reagent alcohol (Approximately 2:8 of apixaban: HPMC 603 weight ratio) to create a thin film. In this example, film was dried under vacuum by Genevac solvent evaporator.

### Example 23

Utilizing procedure described in Example 6, apixaban-HPMCAS-LF- HPMC 603 was prepared by applying a solution containing a known concentration of apixaban, HPMCAS-LF, and HPMC 603 polymer in reagent alcohol (Approximately 2:1.3:6.5 of apixaban: HPMCAS-LF:HPMC 603 weight ratio) to create a thin film. In this example, film was dried under vacuum by Genevac solvent evaporator.

### Example 24

Dissolution testing of examples (example 1-4) and standard examples (example 5) were performed by microdissolution method described by Curatolo et. al. (*Pharm. Res.* 26(6) 1419-1431 2009). For each sample, about 1.5 to 3 mg was weighed and placed in a centrifuge tube. Then, 1.5 mL of dissolution solution (pH 1.2 0.1N hydrochloric acid solution or pH 6.8 phosphate buffer solution) was added to the tube and vortexed for one minute (non-sink conditions, which refers to the drug loading concentration is higher than solubility of drug alone in the dissolution media). The tube was placed in a centrifuge and after 6 minutes, the solution was centrifuged for one minute. The aliquot (25 to 50 µL) was removed and collected into a HPLC vial. For each time point (0, 5, 10, 15, 20, 30, 60, 90 and 120 minutes for dissolution test conducted at pH 1.2 and 0, 5, 10, 20, 30, 60, 90, 120 and 180 minutes for dissolution test conducted at pH 6.8) sample was collected. Each sample was diluted 1:1 with acetonitrile. Following sample collection and preparation, prasugrel concentration in the dissolution solution was determined by using an HPLC (Agilent 100 series HPLC, Agilent, Santa Clara, CA) with Zorbax SB-C8 column with absorbance measured at 254 nm with a UV spectrophotometer.

### Example 25

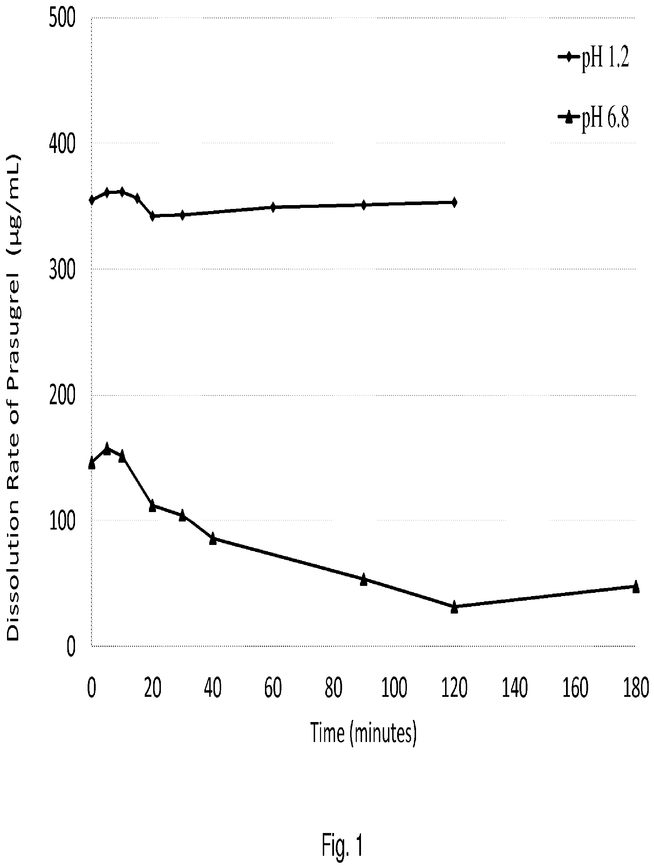

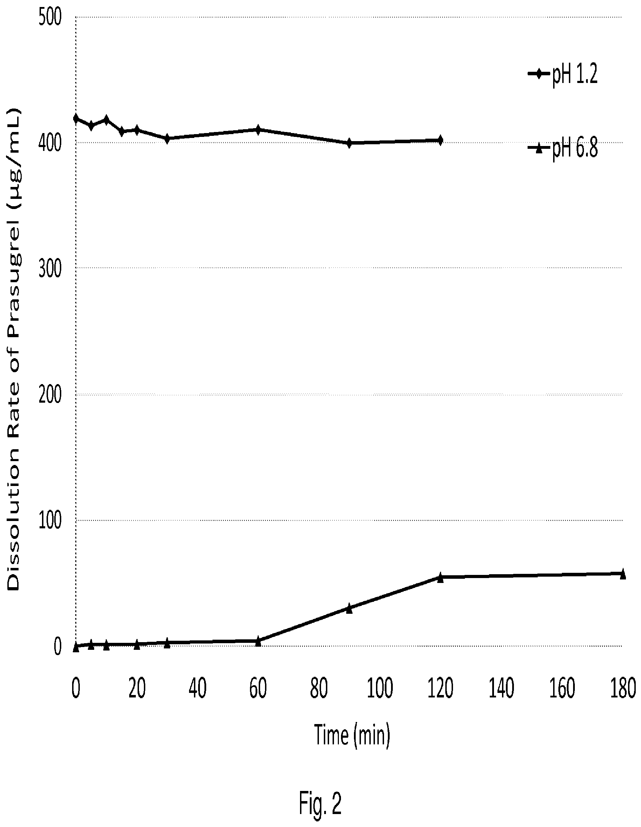

Presugrel is a poorly water soluble weakly basic compound (pKa=5.48) with a highly pH dependent solubility profile; the aqueous solubility in water is very low (0.00237 mg/mL) (Source: Drugbank). Results of prasugrel dissolution testing is shown in Figure 1-4 for Example 1-5. Shown in Fig. 1 (Example 1) and 2 (Example 2), dissolution of prasugrel from solid dispersions of prasugrel with a water soluble polymer (HPMC-Fig 1 and Soluplus^{®}-Fig 2) in two different pH solutions of 1.2 and 6.8 are widely different. The difference in prasugrel dissolution can be as much as 400 fold between pH 1.2 and 6.8.

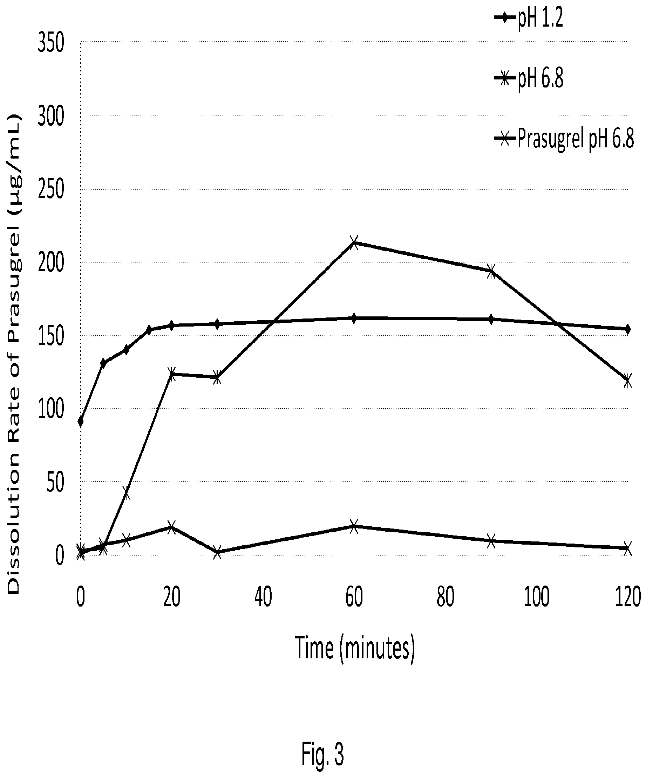

When the formulation consists of the same API with both a water-soluble polymer (Soluplus^{®}) and enteric polymer (HPMCAS-LF), the difference in dissolution at pH 1.2 and 6.8 surprisingly becomes much less after 90 minutes of dissolution, as shown in Fig. 3 (Example 3), Also, there is almost 20-30 fold improvement with the fused amorphous prasugrel solubility at pH 6.8.

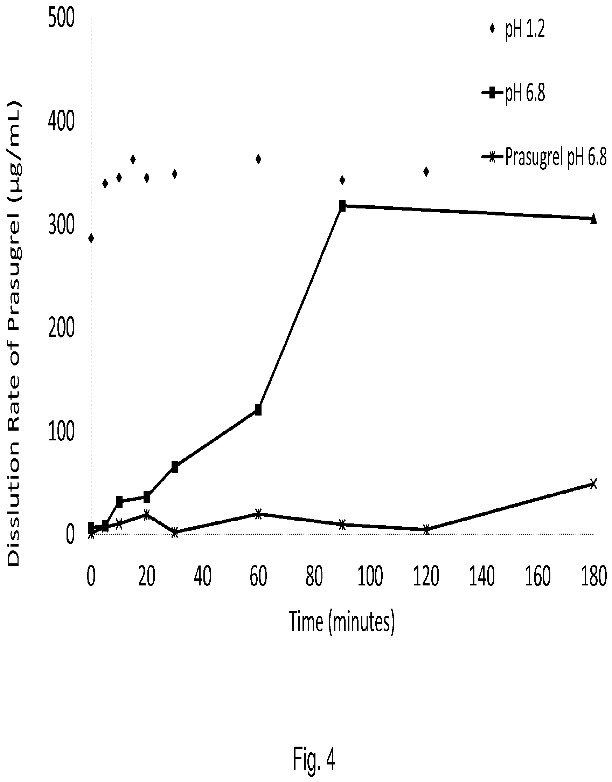

With Example 4 (Fig. 4), when the formulation consists of the same API with HPMC 603, Soluplus^{®}, and enteric polymer (HPMCAS-LF), after 90 minutes, the difference in prasugrel dissolution at pH 1.2 and pH 6.8 is dramatic improved as shown in Fig. 4. Comparing the prasugrel dissolution from solid dispersions to the fused amorphous polymer show at least a 30-fold improvement in pH 6.8 after 90 minutes from beginning.

### Example 26

Dissolution testing of examples (Example 6-7) were performed by microdissolution method described in Example 24. The total drug loading in dissolution medium is 2 mg/mL (non-sink conditions). Clopidogrel concentration in the dissolution solution was determined by using an HPLC (Agilent 100 series HPLC, Agilent, Santa Clara, CA) with Shinwa Ultron ES-OVM, 5 µm, column with absorbance measured at 220 nm with a UV spectrophotometer. For comparative purpose, dissolution testing of examples (Example 8-9) were also performed by microdissolution method described in Example 20 and tested by the same HPLC method. The total drug loading in dissolution medium is 0.1 mg/mL (non-sink conditions) due to very low solubility achieved by these comparative formulations.

### Example 27

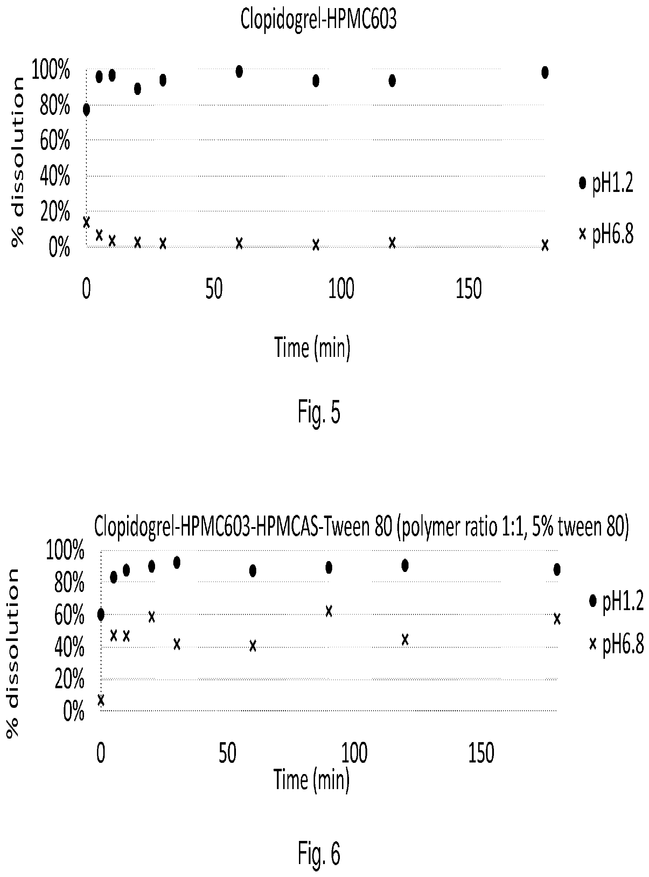

Clopidogrel is a poorly water soluble weakly basic compound (pKa=4.5) with a highly pH dependent solubility profile (Source: FDA). The determined aqueous solubility at pH 6.8 is very low (0.014 mg/mL), whereas its solubility at pH 1.2 is 5.2 mg/mL. Results of clopidogrel dissolution testing is shown in Figure 5-6 for Example 6-7.

Shown in Fig. 5 (Example 6), dissolution of clopidogrel from solid dispersions with one water soluble polymer (HPMC) in two different pH solutions are widely different, with 1.3% (0.026 mg/mL) of drug dissolved at pH 6.8 vs 93.6% (1.87 mg/mL) dissolved at pH 1.2 at 90-minute time point. The difference in drug dissolution can be as much as 72 fold at the time point. Only less than two fold of solubility improvement by solid dispersion with HPMC over that of drug alone at pH 6.8 was observed.

When the formulation consists of the same API with both water-soluble polymer (HPMC) and enteric polymer (HPMCAS-LF) and surfactant (Tween 80), not only the difference in dissolution at pH 1.2 (89.2%, 1.78 mg/mL, dissolved) and pH 6.8 (62.4%, 1.24 mg/mL, dissolved) surprisingly becomes less than 1.5 fold at 90 minutes, as shown in Fig. 6 (Example 7); but also at 90 minutes time point, there is almost 48 fold improvement over clopidogrel solid dispersion with HPMC, and 89 fold improvement over solubility of clopidogrel alone at pH 6.8.

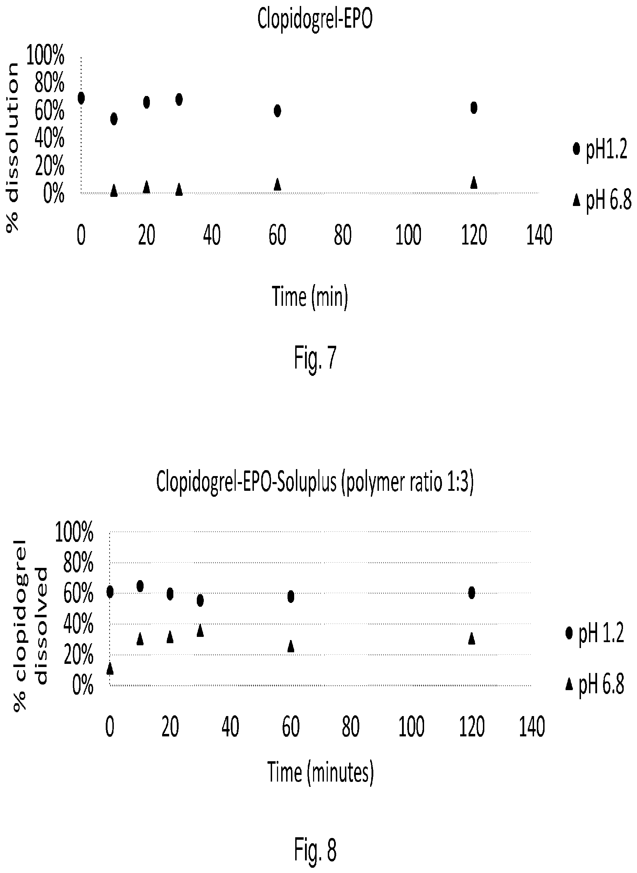

For comparative purpose (Example 8-9), clopidogrel (a weakly basic compound) solid dispersion with gastric-soluble polymer-Eudragit EPO and/or water-soluble polymer, Soluplus^{®} were also prepared and tested. Due to reduced solubility in this composition, total target drug concentration is only 0.1 mg/mL for Example 8-9 vs 2 mg/mL in example 6-7. Shown in Fig. 7 (clopidogrel:EPO=2:8) (Example 8) and Fig. 8 (clopidogrel:EPO:Soluplus^{®}=2:2:6) (Example 9), not only the difference in dissolution at pH 1.2 and pH 6.8 were widely different at 90 minutes for both formulation; but also at 90 minutes time point, there is a reduction in solubility for Example 8 (0.0084 mg/mL) and essentially no improvement for Example 9 (0.03 mg/mL) as compared to the solubility of clopidogrel alone at pH 6.8 (0.014 mg/mL).

### Example 28

Dissolution testing of examples (Example 10-13) were performed by microdissolution method described in Example 24. The drug loading in the dissolution medium is 0.1 mg/mL (non-sink conditions). Following sample collection and preparation, diclofenac concentration in the dissolution solution was determined by using an HPLC (Agilent 100 series HPLC, Agilent, Santa Clara, CA) with Synergi Polar-RP column with absorbance measured at 272 nm with a UV spectrophotometer.

### Example 29

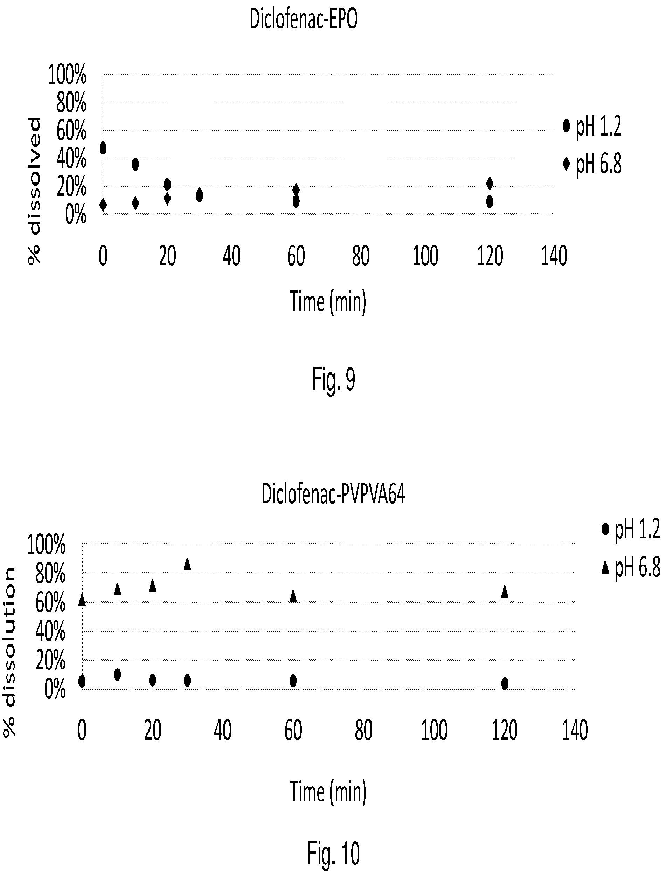

Diclofenac is a poorly water-soluble, weakly acidic compound (pKa=4.15) with aqueous solubility of 2.37 µg/mL in water with a highly pH dependent solubility profile (Source: Drugbank). Its solubility is every low at low pH and increases with increasing pH. Results of diclofenac dissolution testing is shown in Figure 9-12 for Example 10-13.

Shown in Fig. 9 (Example 10) and 10 (Example 11), both solid dispersions with either gastric-soluble polymer (Eudragit E (EPO)-Fig 9 or water-soluble polymer PVPVA 64-Fig 10 did not change pH-dependent dissolution profile of diclofenac, showing lower level of dissolution at pH 1.2 than pH 6.8. Slight improvement in dissolution at pH 1.2 was observed on Eudraigt E solid dispersion, however it fell (~10% (10 µg/mL) below the level of pH 6.8 within 20 minutes. In addition, the dissolution of ibuprofen at pH 6.8 was suppressed by solid dispersion (Fig 10) due to the insolubility of Eudragit E at pH 6.8.

Shown in Fig 10, for Solid dispersion made with PVPVA-64, the dissolution of diclofenac from the formulation in the two different pH solutions of 1.2 and 6.8 are still widely different. The difference in dicolfenac dissolution can be as much as 11 fold between pH 6.8 and pH 1.2 (Fig 10). Enhancement of dissolution by single polymer at pH 1.2 is only marginal about 3-4 folds over solubility of diclofenac alone in water.

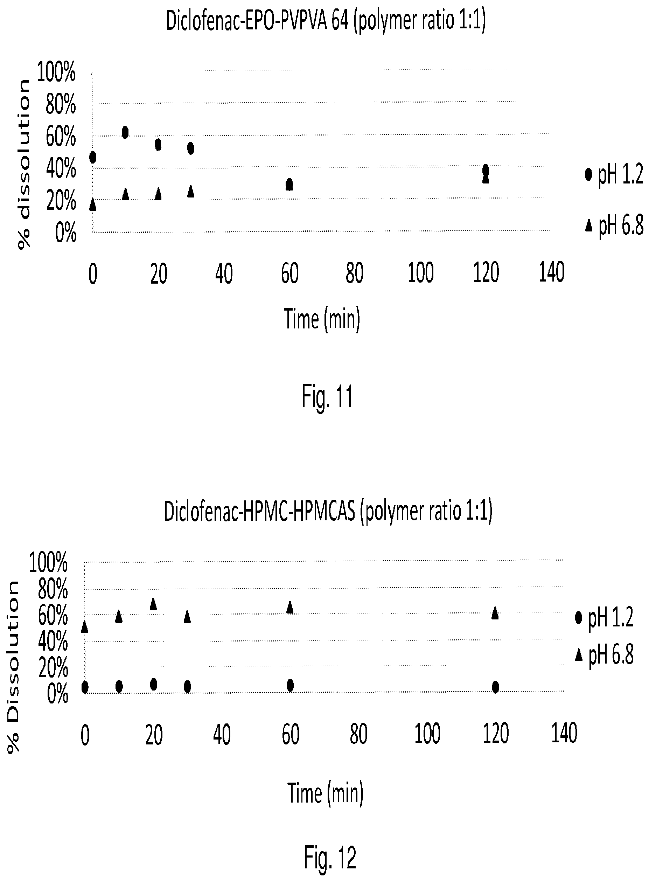

When the formulation consists of the same API with both water-soluble polymer (PVPVA-64) and gastric-soluble polymer (Eudragit E), not only the difference in dissolution at pH 1.2 and 6.8 becomes closer at steady state, as shown in Fig. 11 (Example 12) , but also, there is almost 13 fold improvement over the solubility of diclofenace alone at pH 1.2.

For comparative purpose, diclofenac (a weakly acidic compound) solid dispersion with enteric polymer-HPMCAS_LF and water-soluble polymer, HPMC 603 were also prepared and tested. Shown in Fig. 12 (diclofenac:HPMCAS:HPMC=2:4:4) (Example 13), not only the difference in dissolution at pH 1.2 and pH 6.8 were widely different at 60-120 minutes; but also there is no enhancement in solubility (6 µg/mL) as compared to solubility of diclofenac alone at pH 1.2.

### Example 30

Dissolution testing of examples (Example 14-20) were performed by microdissolution method described in Example 24. The drug loading in the dissolution medium is 2 mg/mL (non-sink condition). Following sample collection and preparation, ibuprofen concentration in the dissolution solution was determined by using an HPLC (Agilent 100 series HPLC, Agilent, Santa Clara, CA) with Synergi Polar RP C18, 4 µm column with absorbance measured at 254 nm with a UV spectrophotometer.

### Example 31

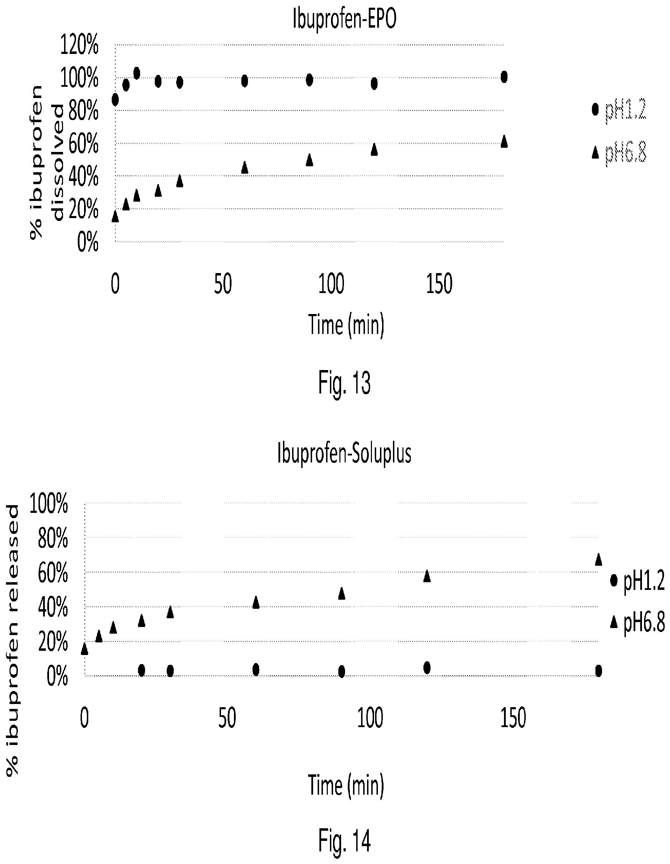

Ibuprofen is a poorly water-soluble, weakly acidic compound (pKa=4.85) with low aqueous solubility of 21 µg/mL in water with a highly pH dependent solubility profile (Source: Drugbank). Its solubility is every low at low pH and increases with increasing pH. Results of ibuprofen dissolution testing is shown in Figure 13-19 for Example 14-20.

Shown in Fig. 13 (Example 14), even though the dissolution of ibuprofen at pH 1.2 from solid dispersions with one gastric-soluble polymer (Eudragit E-Fig 13) was significantly enhanced, dissolution of ibuprofen in two different pH solutions (pH 6.8 and 1.2) are still widely different.

Shown in Fig. 14 (Example 15), dissolution of ibuprofen from solid dispersions with one water soluble polymer (Soluplus-Fig 14) in two different pH solutions (pH 1,2 and 6.8) are also widely different without significant improvement in dissolution at pH 1.2. The difference in ibuprofen dissolution at pH 1.2 and 6.8 can be as much as 18 fold for Soluplus solid dispersion (Fig 14) at 90 minute time point.

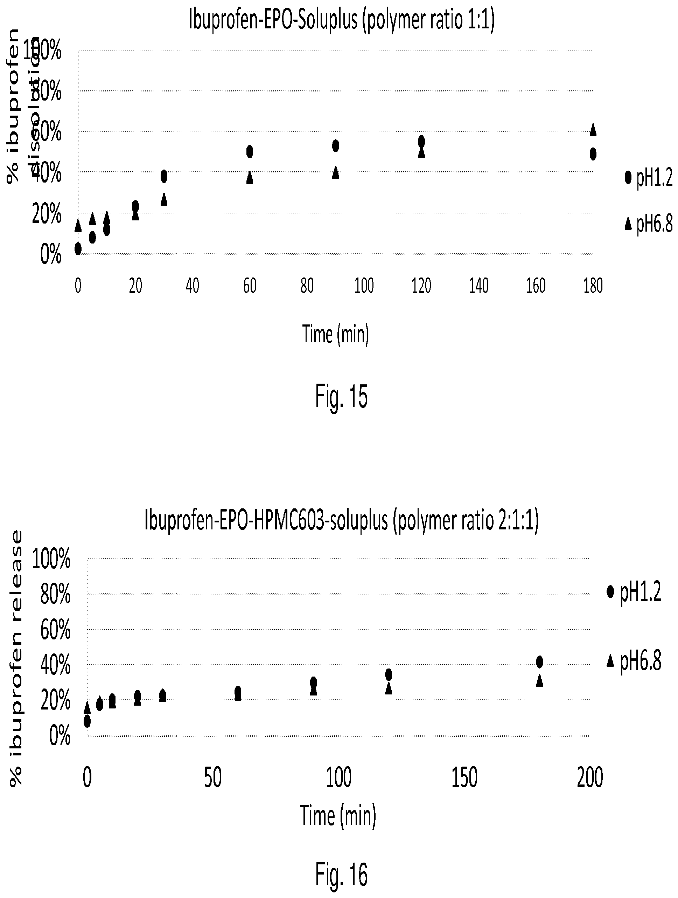

As shown in Fig. 15 (Example 16), when the formulation consists of the same API with both water-soluble polymer (Soluplus) and gastric-soluble polymer (Eudragit E) (1:1 polymer weight ratio), the difference in dissolution at pH 1.2 and 6.8 becomes less than 1.3 fold. Also, there is almost 50 fold improvement in solubility over that of ibuprofen alone at pH 1.2.

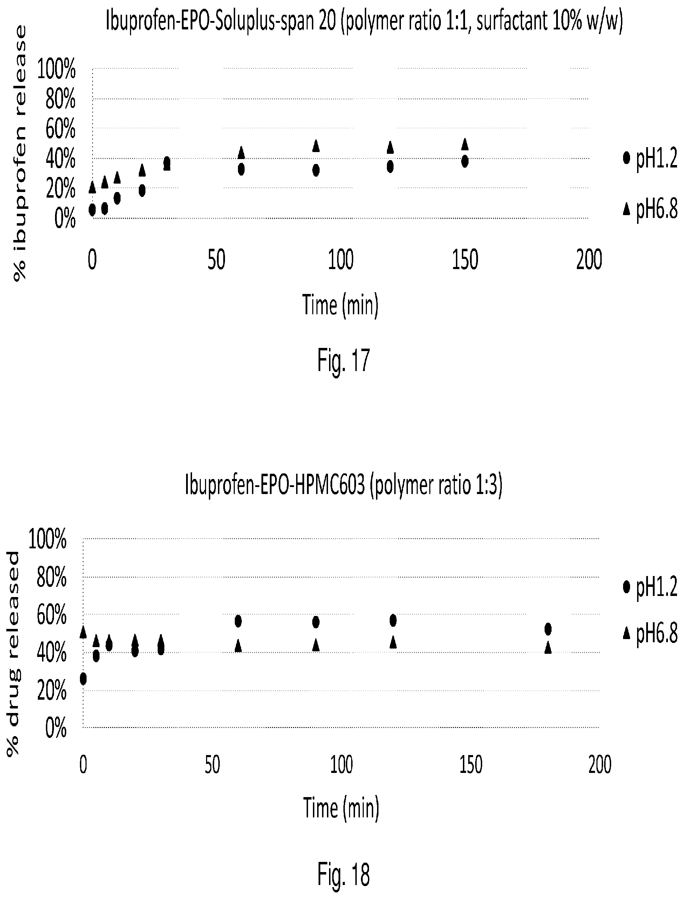

Shown in Example 17 (Fig. 16) and Example 18 (Fig 17), with further addition of soluble polymer (HPMC 603) or surfactant (Span 20) to the solid dispersions, pH-independency of ibuprofen dissolution was maintained with (ratio of amount dissolved at pH 6.8 to pH 1.2 <1.5) (Eudragit E/Soluplus/HPMC (2:1:1 weight ratio)-Fig. 16, Eudragit E/Soluplus/Span 20 (1:1:0.25-Fig 17).

With Example 19 (Fig 18), similar observation in pH-independency of ibuprofen dissolution was found when the formulation consists of the same API with both water-soluble polymer (HPMC) and gastric-soluble polymer (Eudragit E) (Eudragit E: HPMC=1:3 weight ratio). Also, there is almost 53 fold improvement in solubility over that of ibuprofen alone at pH 1.2.

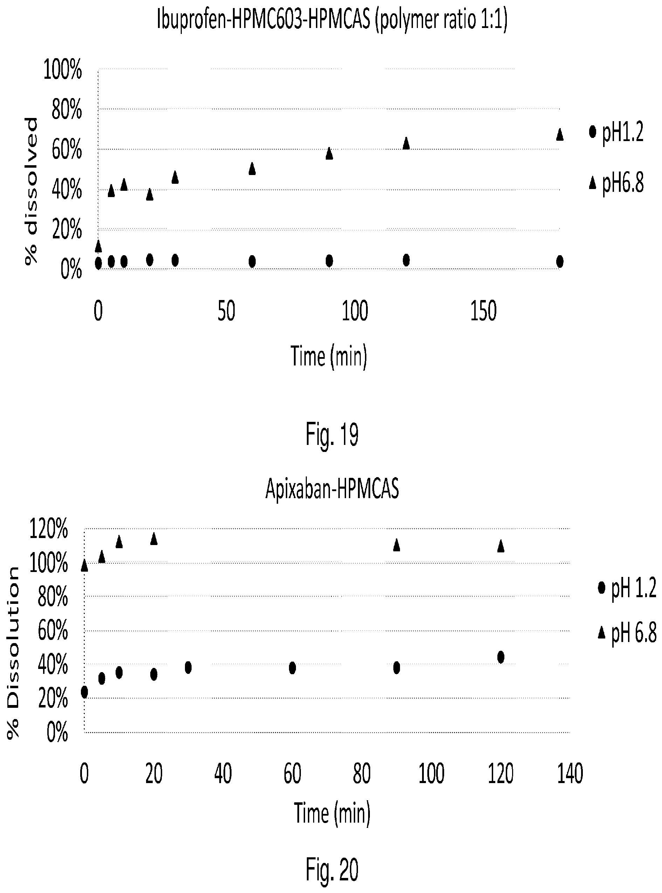

For comparative purpose, ibuprofen (a weakly acidic compound) solid dispersion with enteric polymer-HPMCAS_LF and water-soluble polymer, HPMC 603 were also prepared and tested. Shown in Fig. 19 (Example 20) ibuprofen:HPMCAS:HPMC=2:4:4), not only the difference in dissolution at pH 1.2 and pH 6.8 were widely different at 90 minutes for both formulation; but also at 90 minutes time point, there is essentially no improvement (0.086 mg/mL) as compared to the solubility of ibuprofen alone at pH 1.2 (0.021 mg/mL).

### Example 32

Dissolution testing of examples (Example 21-23) were performed by microdissolution method described in Example 24. The drug loading in the dissolution medium is 0.1 mg/mL (non-sink conditions). Following sample collection and preparation, apixaban concentration in the dissolution solution was determined by using an HPLC (Agilent 100 series HPLC, Agilent, Santa Clara, CA) with Phenomenex Synergi Polar-RP column with absorbance measured at 280 nm with a UV spectrophotometer.

### Example 33

Apixaban is a poorly water-soluble, compound with no detectable pKa (or non-ionizable functional group) within pH range of 0.0-10.0 (Source: Drugbank). It has low aqueous solubility of 40-50 µg/mL in water with a pH in-dependent solubility profile. Its solubility is every low throughout physiological pH range of 1.0-8.0. Results of apixaban dissolution testing is shown in Figure 20-22 for Example 21-23.

Shown in Fig. 20 (Example 21), it was found that the dissolution of apixaban at pH 6.8 was enhanced from solid dispersions with an enteric polymer (HPMCAS-LF-Fig 20). However, dissolution of apixaban in the two pH (pH 1.2 and 6.8) are still widely different with less than 40% dissolved at pH 1.2 at 90 minutes due to enteric nature of HPMCAS polymer.

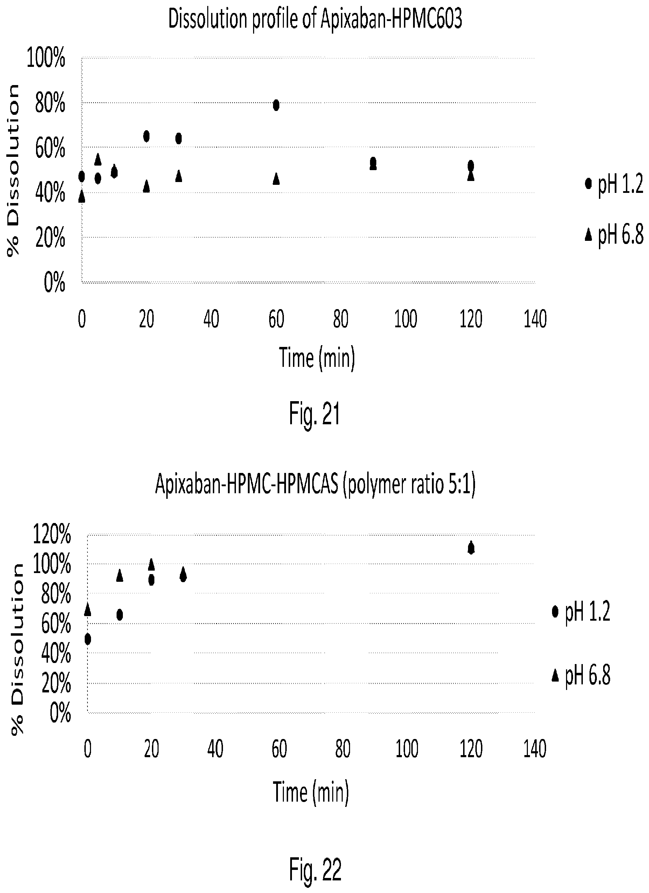

Shown in Fig. 21 (Example 22), as expected to an non-ionizable compound with pH-independent solubility, dissolution of apixaban from solid dispersions with one water-soluble, pH independent polymer (HPMC 603-Fig 21) in the two different pH solutions are similar. However, the extent of dissolution from solid dispersion at both pH of 1.2 and 6.8 was relatively low (less than 55% at 90 minute time point).

Show in Fig 22, surprisingly, when the formulation consists of the same API with both water-soluble polymer (HPMC) and enteric polymer (HPMCAS-LF), not only the difference in dissolution at pH 1.2 and 6.8 becomes less than 1.1 fold, as shown in Fig. 18 (Example 19), but also, there is almost complete and rapid release of apixaban at both pHs.

## Claims

1. A solid dispersion, which comprises:
(I) at least one poorly water-soluble acidic compound (API) with at least one pharmaceutically acceptable water-soluble polymer, at least one gastric-soluble polymer, and at least one pharmaceutically acceptable surfactant,
wherein in the absence of said solid dispersion, the poorly water-soluble acidic compound has at least one pKa (acid) within the range of 0.0-10.0, and a pH-dependent solubility in aqueous environment of pH 1.0-8.0 with the lowest aqueous solubility of <1.0 mg/mL at pH 1.0-2.0,
wherein, in said solid dispersion, said gastric-soluble polymer(s) is dispersed in said solid dispersion matrix with API, water-soluble polymer(s) and/or surfactant(s) at a solid state,
wherein, in said solid dispersion, said polymer(s) and/or surfactant(s) are present in an amount such that the concentration of said dissolved poorly water-soluble acidic compound at both pH 1.0-2.0 and pH 6.0-8.0 at or after 90 minutes time point during non-sink dissolution test in aqueous environment is at least 2 fold of the solubility of said API alone in aqueous environment at pH 1.0-2.0 not containing said solid dispersion,
wherein, in said solid dispersion, said polymer(s) and/or surfactant(s) are present in an amount such that at or after 90 minutes time point after non-sink dissolution test in aqueous environment, the ratio of the concentration of said dissolved poorly water-soluble acidic compound at pH 6.0-8.0 to that at pH 1.0-2.0 is less than 2.0; or
(II) at least one poorly water-soluble neutral or non-ionizable compound (API) with at least one pharmaceutically acceptable water-soluble polymer, at least one pH-sensitive polymer, and at least one pharmaceutically acceptable surfactant,
wherein, in the absence of said solid dispersion, the poorly water-soluble neutral or non-ionizable compound has no detectable (or calculated) pKa within the range of 1.0 to 12.0, and has a pH-independent solubility in aqueous environment of pH 1.0-8.0 with the lowest aqueous solubility of <1.0 mg/mL,
wherein, in said solid dispersion, said pH-sensitive polymer(s) is disposed in said solid dispersion matrix with API, water-soluble polymer(s) and/or surfactant(s) at a solid state,
wherein, in said solid dispersion, said polymer(s) and/or surfactant(s) are present in an amount such that the concentration of said dissolved poorly water-soluble compound at both pH 1.0-2.0 and pH 6.0-8.0 at or after 90 minutes time point during non-sink dissolution test in aqueous environment is at least 2 fold of the solubility of said API alone in aqueous environment at pH 1.0-8.0 not containing said solid dispersion,
wherein, in said solid dispersion, said polymer(s) and/or surfactant(s) are present in an amount such that at or after 90 minutes time point after non-sink dissolution test in aqueous environment, the ratio of the concentration of said dissolved poorly water-soluble compound at pH 6.0-8.0 to that at pH 1.0-2.0 is between 0.5-2.0; and
wherein in (I), and (II), the weight ratio of said water-soluble polymer(s) and the pharmaceutical acceptable surfactant(s) to said pH-sensitive polymer(s) or gastric-soluble polymer(s) is in the range of 0.5:9.5 to 9.5:0.5.

2. The solid dispersion of claim 1, wherein the weight ratio of said water soluble polymer(s) and the pharmaceutical acceptable surfactant(s) to said pH-sensitive polymer(s) or gastric-soluble polymer(s) is in a range from 1:9 to 1:1, 1:1 to 9:1, and 1:2 to 5:1.

3. The solid dispersion of claim 1, wherein said polymer(s) and/or surfactant(s) are present in an amount such that at or after the 90 minutes time point after dissolution in non-sink aqueous environment, the ratio of the concentration of a dissolved poorly water-soluble basic compound at pH 1.0-2.0 to that at pH 6.0-8.0 is less than 1.5, and optionally less than 1.25.

4. The solid dispersion of claim 1, wherein said gastric-soluble is selected from the group consisting of: methacrylic acid copolymers, in particular butylmethacrylat-(2-dimethylaminoethyl)-methacrylat-methylmethacrylat-copolymer (1:2:1), which is a copolymer based on (2-dimethylaminoethyl) methacrylate, butyl methacrylate and methyl methacrylate having a mean molecular weight of about 150,000, chitosan (linear polysaccharide composed of randomly distributed β-(1-4)-linked D-glucosamine (deacetylated unit), and N-acetyl-D-glucosamine (acetylated unit)), or other high molecular weight polymer with cationic functional group, and any combinations thereof.

5. The solid dispersion of claim 1, wherein drug loading of said poorly water-soluble compound is in a range of 0% w/w to 95% w/w.

6. The solid dispersion of claim 1, wherein the weight ratio of the at least one pharmaceutically acceptable surfactant to the at least one pharmaceutically acceptable water-soluble polymer is in a range from about 0.01:9.99 to about 9.99:0.01.

7. The solid dispersion of claim 1, wherein said pharmaceutical compositions of said dispersion are prepared in the following manner:
mechanical mixing of said crystalline API with diameter less than 10 micron with a blend of two or more said polymers and/or said surfactant prepared as a solid dispersion, in particular by spray drying, hot melt extruding, or lyophilizing;
mechanical mixing of said amorphous API with diameter less than 10 micron with a blend of two or more said polymers and/or said surfactant prepared as a solid dispersion, in particular by spray drying, hot melt extruding, or lyophilizing;
dispersion of said API, either crystalline or amorphous state, with diameter less than ten micron within a solid matrix of said two or more polymers and/or said surfactant mixture; or
dispersion of said API, either crystalline or amorphous state, with diameter less than ten micron within a solid matrix of said two or more polymers, with said surfactant added as an external phase.

8. The solid dispersion of claim 1, wherein the at least one pH-sensitive polymer is selected from the group consisting of: methacrylic acid copolymers, in particular, butylmethacrylat-(2-dimethylaminoethyl)-methacrylat-methylmethacrylat-copolymer (1:2:1), which is a copolymer based on (2-dimethyl-aminoethyl)methacrylate, butyl methacrylate and methyl methacrylate having a mean molecular weight of about 150,000), chitosan (linear polysaccharide composed of randomly distributed β-(1-4)-linked D-glucosamine (deacetylated unit) and N-acetyl-D-glucosamine (acetylated unit)), or other high molecular weight polymer with cationic functional group, cellulose acetate phthalate (CAP), hydroxypropyl methylcellulose phthalate (HPMCP-50 or HPMCP-55), alkali-soluble acrylic copolymers, polyvinyl acetate phthalate (PVAP), alginates, Carboxymethyl cellulose (CMC), and mixtures of one or more thereof.

9. The solid dispersion of claim 1, wherein the at least one pharmaceutically acceptable water-soluble polymer is selected from the group consisting of: homopolymers and copolymers of N-vinyl lactams, especially homopolymers and copolymers of N-vinyl pyrrolidone, e.g. polyvinylpyrrolidone (PVP), copolymers of N-vinyl pyrrolidone and vinyl acetate or vinyl propionate, polyvinyl caprolactam-polyvinyl acetate-polyethylene glycol graft copolymer marketed such as block copolymers of ethylene oxide and propylene oxide, also known as polyoxyethylene polyoxypropylene block copolymers or polyoxyethylene polypropyleneglycol, such as Poloxamer, lauroyl polyoxyglycerides cellulose esters and cellulose ethers; in particular methylcellulose, hydroxyalkylcelluloses, in particular hydroxypropylcellulose, hydroxyalkylalkylcelluloses, in particular hydroxypropylmethylcellulose, high molecular polyalkylene oxides such as polyethylene
oxide and polypropylene oxide and copolymers of ethylene oxide and propylene oxide, vinyl acetate polymers such as copolymers of vinyl acetate and crotonic acid, partially hydrolyzed polyvinyl acetate (also referred to as partially saponified "polyvinyl alcohol"), polyvinyl alcohol, oligo- and polysaccharides such as carrageenans, galactomannans and xanthan gum, and mixtures of one or more thereof.

10. The solid dispersion of claim 1,
wherein the solid dispersion comprises at least one pharmaceutically acceptable surfactant, and
wherein said at least one pharmaceutically acceptable surfactant is selected from the group consisting of: polyoxyethylene alkyl ethers, polyoxyethylene alkylaryl ethers, polyethylene glycol fatty acid esters, alkylene glycol fatty acid mono esters, sucrose fatty acid esters, sorbitan fatty acid mono esters lauroyl polyoxyglycerides, polyvinyl caprolactam-polyvinyl acetate-polyethylene glycol graft copolymer marketed such as sodium docusate, polyethylene glycol-26 glycerin, polyoxyethylene monostearate, d-α-Tocopheryl polyethylene glycol 1000 succinate (vitamin E TPGS), polyoxyethylene alkyl ethers, polyethylene glycol fatty acid esters, alkylene glycol fatty acid mono esters, sucrose fatty acid esters, sorbitan fatty acid mono esters, sorbitan stearate, polyoxyethylene castor oil derivatives, or polyoxyethyleneglycerol oxystearate or block copolymers of ethylene oxide and propylene oxide, also known as polyoxyethylene polyoxypropylene block copolymers or polyoxyethylene polypropyleneglycol, such as Poloxamer or a mono fatty acid ester of polyoxyethylene sorbitan, e.g. polyoxyethylene sorbitan monooleate, and mixtures of one or more thereof.

11. A method to prepare a pharmaceutical dosage form for oral administration to a mammal using said solid dispersion according to claim 1.

12. The solid dispersion of claim 1, wherein the API is the water-soluble acidic compound.

13. The solid dispersion of claim 1, wherein the API is the poorly water-soluble neutral or non-ionizable compound.

14. The solid dispersion of claim 3, wherein, at least one of said poorly water-soluble basic compound (API) is Prasugrel or Clopidogrel;
wherein, in the absence of said solid dispersion, the poorly water-soluble basic compound has at least one pKa (base) within the range of 0.0-10.0, and a pH- dependent solubility in aqueous environment of pH 1.0-8.0 with the lowest aqueous solubility of <1.0 mg/mL at pH 6.0-8.0;
wherein, in said solid dispersion, said gastric-soluble polymer(s) is dispersed in a solid dispersion matrix with API, water-soluble polymer(s) and/or surfactant(s) at a solid state; wherein the gastric-soluble polymer is a hydroxypropyl methylcellulose acetate succinate; wherein said water-soluble polymer is selected from the group consisting of polyvinyl caprolactam-polyvinyl acetate-polyethylene glycol graft copolymer hydroxypropylcellulose, and hydroxypropylmethylcelluloses, and
said surfactant is polyoxyethylene sorbitan monooleate, polyoxyethylene sorbitan monostearate, or polyoxyethylene sorbitan monopalmitate.

15. The solid dispersion of claim 1, wherein, at least one said poorly water-soluble acidic compound (API) is selected from the group consisting of diclofenac or ibuprofen;
wherein, in the absence of said solid dispersion, the poorly water-soluble acidic compound has at least one pKa (acid) within the range of 0.0-10.0, and a pH- dependent solubility in aqueous environment of pH 1.0-8.0 with the lowest aqueous solubility of <1.0 mg/mL at pH 1.0-2.0;
wherein, in said solid dispersion, said gastric-soluble polymer(s) is dispersed in a solid dispersion matrix with API, water-soluble polymer(s) and/or surfactant(s) at a solid state; wherein the gastric-soluble polymer is a hydroxypropyl methylcellulose acetate succinate;
wherein said water-soluble polymer is selected from the group consisting of polyvinyl caprolactam-polyvinyl acetate-polyethylene glycol graft copolymer hydroxypropylcellulose, and hydroxypropylmethylcelluloses, and
said surfactant is polyoxyethylene sorbitan monooleate, polyoxyethylene sorbitan monostearate, or polyoxyethylene sorbitan monopalmitate.

## Patentansprüche

1. Feste Dispersion, die Folgendes umfasst:
(I) mindestens eine schwer wasserlösliche saure Verbindung (API) mit mindestens einem pharmazeutisch verträglichen wasserlöslichen Polymer, mindestens einem magenlöslichen Polymer und mindestens einem pharmazeutisch verträglichen oberflächenaktivem Mittel,
wobei in Abwesenheit der festen Dispersion die schwer wasserlösliche saure Verbindung mindestens einen pKs-Wert (Säure) im Bereich von 0,0-10,0 und eine pH-abhängige Löslichkeit in wässriger Umgebung von pH 1,0-8,0 aufweist, wobei die geringste wässrige Löslichkeit <1,0 mg/ml bei pH 1,0-2,0 beträgt,
wobei in der festen Dispersion das magensaftlösliche Polymer bzw. die magensaftlöslichen Polymere in der festen Dispersionsmatrix mit API, dem wasserlöslichen Polymer bzw. den wasserlöslichen Polymeren und/oder dem oberflächenaktiven Mittel bzw. den oberflächenaktiven Mitteln in einem festen Zustand dispergiert sind,
wobei in der festen Dispersion das (die) Polymer(e) und/oder das (die) oberflächenaktiven Mittel in einer solchen Menge vorhanden ist (sind), dass die Konzentration der gelösten schwer wasserlöslichen sauren Verbindung sowohl bei pH 1,0-2,0 als auch bei pH 6,0-8,0 zum oder nach dem 90-Minuten-Zeitpunkt während des Nicht-Sink-Auflösungstests in wässriger Umgebung mindestens das Zweifache der Löslichkeit der API allein in wässriger Umgebung bei pH 1,0-2,0 beträgt, die nicht die feste Dispersion enthält,
wobei in der festen Dispersion das (die) Polymer(e) und/oder das (die) oberflächenaktiven Mittel in einer solchen Menge vorhanden ist (sind), dass zum oder nach dem 90-Minuten-Zeitpunkt nach dem Nicht-Sink-Auflösungstest in wässriger Umgebung das Verhältnis der Konzentration der gelösten schwer wasserlöslichen sauren Verbindung bei pH 6,0-8,0 zu der bei pH 1,0-2,0 weniger als 2,0 beträgt; oder
(II) mindestens eine schwer wasserlösliche neutrale oder nicht ionisierbare Verbindung (API) mit mindestens einem pharmazeutisch verträglichen wasserlöslichen Polymer, mindestens einem pH-empfindlichen Polymer und mindestens einem pharmazeutisch verträglichen oberflächenaktiven Mittel,
wobei in Abwesenheit der festen Dispersion die schwer wasserlösliche neutrale oder nicht-ionisierbare Verbindung keinen nachweisbaren (oder berechneten) pKs innerhalb des Bereichs von 1,0 bis 12,0 hat und eine pHunabhängige Löslichkeit in wässriger Umgebung von pH 1,0-8,0 mit der niedrigsten wässrigen Löslichkeit von <1,0 mg/ml aufweist,
wobei in der festen Dispersion das (die) pH-empfindliche(n) Polymer(e) in der festen Dispersionsmatrix mit API, wasserlöslichem(n) Polymer(en) und/oder oberflächenaktivem(n) Mittel(n) in einem festen Zustand angeordnet ist (sind),
wobei in der festen Dispersion das (die) Polymer(e) und/oder das (die) oberflächenaktiven Mittel in einer solchen Menge vorhanden ist (sind), dass die Konzentration der gelösten, schwer wasserlöslichen Verbindung sowohl bei pH 1,0-2,0 als auch bei pH 6,0-8,0 zum oder nach dem 90-Minuten-Zeitpunkt während des Nicht-Sink-Auflösungstests in wässriger Umgebung mindestens das Zweifache der Löslichkeit der API allein in wässriger Umgebung bei pH 1,0-8,0 beträgt, die nicht die feste Dispersion enthält,
wobei in der festen Dispersion das (die) Polymer(e) und/oder das (die) oberflächenaktiven Mittel in einer solchen Menge vorhanden ist (sind), dass zum oder nach dem 90-Minuten-Zeitpunkt nach dem Nicht-Sink-Auflösungstest in wässriger Umgebung das Verhältnis der Konzentration der gelösten, schwer wasserlöslichen Verbindung bei pH 6,0-8,0 zu der bei pH 1,0-2,0 zwischen 0,5-2,0 liegt; und
wobei in (I) und (II) das Gewichtsverhältnis des (der) wasserlöslichen Polymers (Polymere) und des (der) pharmazeutisch verträglichen oberflächenaktiven Mittel(s) zu dem (den) pH-empfindlichen Polymer(en) oder dem (den) magenlöslichen Polymer(en) im Bereich von 0,5:9,5 bis 9,5:0,5 liegt.

2. Feste Dispersion nach Anspruch 1, wobei das Gewichtsverhältnis des(der) wasserlöslichen Polymer(s)(e) bzw. der wasserlöslichen Polymere und des pharmazeutisch verträglichen oberflächenaktiven Mittels bzw. der pharmazeutisch verträglichen oberflächenaktiven Mittel zu dem pH-empfindlichen Polymer bzw. den pH-empfindlichen Polymeren oder dem magenlöslichen Polymer bzw. den magenlöslichen Polymeren in einem Bereich von 1:9 bis 1:1, 1:1 bis 9:1 und 1:2 bis 5:1 liegt.

3. Feste Dispersion nach Anspruch 1, wobei das/die Polymer(e) und/oder das/die oberflächenaktiven Mittel in einer solchen Menge vorhanden ist/sind, dass zu oder nach dem 90-Minuten-Zeitpunkt nach der Auflösung in einer nicht-sinkenden wässrigen Umgebung das Verhältnis der Konzentration einer gelösten, schwer wasserlöslichen basischen Verbindung bei pH 1,0-2,0 zu der bei pH 6,0-8,0 weniger als 1,5 und wahlweise weniger als 1,25 beträgt.

4. Feste Dispersion nach Anspruch 1, wobei die magenlösliche Substanz ausgewählt ist aus der Gruppe bestehend aus: Methacrylsäure-Copolymeren, insbesondere Butylmethacrylat-(2-Dimethylaminoethyl)-methacrylat-Methylmethacrylat-Copolymer (1:2:1), das ein Copolymer auf Basis von (2-Dimethylaminoethyl)methacrylat, Butylmethacrylat und Methylmethacrylat mit einem mittleren Molekulargewicht von etwa 150.000 ist, Chitosan (lineares Polysaccharid, das aus zufällig verteiltem β-(1-4)-verknüpftem D-Glucosamin (deacetylierte Einheit) und N-Acetyl-D-Glucosamin (acetylierte Einheit) besteht) oder einem anderen hochmolekularen Polymer mit einer kationischen funktionellen Gruppe und beliebigen Kombinationen davon.

5. Feste Dispersion nach Anspruch 1, wobei die Wirkstoffbeladung der schwer wasserlöslichen Verbindung in einem Bereich von 0 Gew.-% bis 95 Gew.-% liegt.

6. Feste Dispersion nach Anspruch 1, wobei das Gewichtsverhältnis des mindestens einen pharmazeutisch verträglichen oberflächenaktiven Mittels zu dem mindestens einen pharmazeutisch verträglichen wasserlöslichen Polymer in einem Bereich von etwa 0,01:9,99 bis etwa 9,99:0,01 liegt.

7. Feste Dispersion nach Anspruch 1, wobei die pharmazeutischen Zusammensetzungen der Dispersion auf folgende Weise hergestellt werden:
mechanisches Mischen des kristallinen API mit einem Durchmesser von weniger als 10 Mikrometern mit einer Mischung aus zwei oder mehr der Polymere und/oder des oberflächenaktiven Mittels, die als feste Dispersion hergestellt wird, insbesondere durch Sprühtrocknung, Heißschmelzextrusion oder Lyophilisierung;
mechanisches Mischen des amorphen API mit einem Durchmesser von weniger als 10 Mikrometern mit einer Mischung aus zwei oder mehr der Polymere und/oder des oberflächenaktiven Mittels, die als feste Dispersion hergestellt wird, insbesondere durch Sprühtrocknung, Heißschmelzextrusion oder Lyophilisierung;
Dispersion des API in kristallinem oder amorphem Zustand mit einem Durchmesser von weniger als zehn Mikron in einer festen Matrix aus den zwei oder mehr Polymeren und/oder der Mischung oberflächenaktiver Mittel; oder
Dispersion der API, entweder im kristallinen oder amorphen Zustand, mit einem Durchmesser von weniger als zehn Mikron in einer festen Matrix aus den zwei oder mehr Polymeren, wobei das oberflächenaktiven Mittel als äußere Phase hinzugefügt wird.

8. Feste Dispersion nach Anspruch 1, wobei das mindestens eine pH-empfindliche Polymer ausgewählt ist aus der Gruppe bestehend aus: Methacrylsäure-Copolymeren, insbesondere Butylmethacrylat-(2-Dimethylaminoethyl)-methacrylat-Methylmethacrylat-Copolymer (1:2:1), das ein Copolymer auf Basis von (2-Dimethylaminoethyl)methacrylat, Butylmethacrylat und Methylmethacrylat mit einem mittleren Molekulargewicht von etwa 150.000 ist), Chitosan (lineares Polysaccharid, das aus zufällig verteiltem β-(1-4)-verknüpftem D-Glucosamin (deacetylierte Einheit) und N-Acetyl-D-Glucosamin (acetylierte Einheit) besteht), oder einem anderen hochmolekularen Polymer mit kationischer funktioneller Gruppe, Celluloseacetatphthalat (CAP), Hydroxypropylmethylcellulosephthalat (HPMCP-50 oder HPMCP-55), alkalilöslichen Acrylcopolymeren, Polyvinylacetatphthalat (PVAP), Alginaten, Carboxymethylcellulose (CMC) und Mischungen aus einem oder mehreren davon.

9. Feste Dispersion nach Anspruch 1, wobei das mindestens eine pharmazeutisch verträgliche wasserlösliche Polymer ausgewählt ist aus der Gruppe bestehend aus: Homopolymeren und Copolymeren von N-Vinyllactamen, insbesondere Homopolymeren und Copolymeren von N-Vinylpyrrolidon, z. B. Polyvinylpyrrolidon (PVP), Copolymere aus N-Vinylpyrrolidon und Vinylacetat oder Vinylpropionat, Polyvinylcaprolactam-Polyvinylacetat-Polyethylenglykol-Pfropfcopolymere, die wie Blockcopolymere aus Ethylenoxid und Propylenoxid vertrieben werden, auch bekannt als Polyoxyethylen-Polyoxypropylen-Blockcopolymere oder Polyoxyethylen-Polypropylenglykol, wie Poloxamer, Lauroylpolyoxyglyceride, Celluloseester und Celluloseether; insbesondere Methylcellulose, Hydroxyalkylcellulosen, insbesondere Hydroxypropylcellulose, Hydroxyalkylalkylcellulosen, insbesondere Hydroxypropylmethylcellulose, hochmolekulare Polyalkylenoxide wie Polyethylen Oxid und Polypropylenoxid und Copolymere von Ethylenoxid und Propylenoxid, Vinylacetatpolymere wie Copolymere von Vinylacetat und Crotonsäure, teilweise hydrolysiertes Polyvinylacetat (auch als teilweise verseifter "Polyvinylalkohol" bezeichnet), Polyvinylalkohol, Oligo- und Polysaccharide wie Carrageenane, Galactomannane und Xanthangummi sowie Mischungen aus einem oder mehreren davon.

10. Feste Dispersion nach Anspruch 1,
wobei die feste Dispersion mindestens ein pharmazeutisch verträgliches oberflächenaktives Mittel enthält, und
wobei das mindestens eine pharmazeutisch verträgliche oberflächenaktive Mittel ausgewählt ist aus der Gruppe bestehend aus: Polyoxyethylenalkylethern, Polyoxyethylenalkylarylethern, Polyethylenglykolfettsäureestern, Alkylenglykolfettsäuremonoestern, Saccharosefettsäureestern, Sorbitanfettsäuremonoestern, Lauroylpolyoxyglyceriden, Polyvinylcaprolactam-Polyvinylacetat-Polyethylenglykol-Pfropfcopolymer, das wie Natriumdocusat vermarktet wird, Polyethylenglykol-26-Glycerin, Polyoxyethylenmonostearat, d-α-Tocopheryl-Polyethylenglykol-1000-succinat (Vitamin E TPGS), Polyoxyethylenalkylether, Polyethylenglykolfettsäureester, Alkylenglykolfettsäuremonoester, Saccharosefettsäureester, Sorbitanfettsäuremonoester, Sorbitanstearat, Polyoxyethylenrizinusölderivate, oder Polyoxyethylenglycerinoxystearat oder Blockcopolymere von Ethylenoxid und Propylenoxid, auch bekannt als Polyoxyethylen-Polyoxypropylen-Blockcopolymere oder Polyoxyethylen-Polypropylenglykol, wie Poloxamer oder ein Monofettsäureester von Polyoxyethylensorbitan, z.z. B. Polyoxyethylensorbitanmonooleat, und Mischungen aus einem oder mehreren davon.

11. Verfahren zur Herstellung einer pharmazeutischen Dosierungsform zur oralen Verabreichung an ein Säugetier unter Verwendung der festen Dispersion nach Anspruch 1.

12. Feste Dispersion nach Anspruch 1, wobei die API die wasserlösliche saure Verbindung ist.

13. Feste Dispersion nach Anspruch 1, wobei die API die schwer wasserlösliche neutrale oder nicht ionisierbare Verbindung ist.

14. Feste Dispersion nach Anspruch 3, wobei mindestens eine der schwer wasserlöslichen basischen Verbindungen (API) Prasugrel oder Clopidogrel ist;
wobei die schwer wasserlösliche basische Verbindung in Abwesenheit der festen Dispersion mindestens einen pKs-Wert (Base) im Bereich von 0,0-10,0 und eine pH-abhängige Löslichkeit in wässriger Umgebung von pH 1,0-8,0 aufweist, wobei die geringste wässrige Löslichkeit <1,0 mg/ml bei pH 6,0-8,0 beträgt;
wobei in der festen Dispersion das (die) magenlösliche(n) Polymer(e) in einer festen Dispersionsmatrix mit API, wasserlöslichem(n) Polymer(en) und/oder oberflächenaktiven Mittel(n) in einem festen Zustand dispergiert ist (sind);
wobei das magenlösliche Polymer ein Hydroxypropylmethylcelluloseacetatsuccinat ist; wobei das wasserlösliche Polymer ausgewählt ist aus der Gruppe bestehend aus Polyvinylcaprolactam-Polyvinylacetat-Polyethylenglykol-Pfropfcopolymer, Hydroxypropylcellulose und Hydroxypropylmethylcellulosen, und
das oberflächenaktiven Mittel Polyoxyethylen-Sorbitan-Monooleat, Polyoxyethylen-Sorbitan-Monostearat oder Polyoxyethylen-Sorbitan-Monopalmitat ist.

15. Feste Dispersion nach Anspruch 1, wobei mindestens eine der schwer wasserlöslichen sauren Verbindungen (API) ausgewählt ist aus der Gruppe bestehend aus Diclofenac oder Ibuprofen;
wobei in Abwesenheit der festen Dispersion die schwer wasserlösliche saure Verbindung mindestens einen pKs-Wert (Säure) im Bereich von 0,0-10,0 und eine pH-abhängige Löslichkeit in wässriger Umgebung von pH 1,0-8,0 aufweist, wobei die geringste wässrige Löslichkeit <1,0 mg/ml bei pH 1,0-2,0 beträgt;
wobei in der festen Dispersion das (die) magenlösliche(n) Polymer(e) in einer festen Dispersionsmatrix mit API, wasserlöslichem(n) Polymer(en) und/oder oberflächenaktive Mittel(n) in einem festen Zustand dispergiert ist (sind);
wobei das magenlösliche Polymer ein Hydroxypropylmethylcelluloseacetatsuccinat ist; wobei das wasserlösliche Polymer ausgewählt ist aus der Gruppe bestehend aus Polyvinylcaprolactam-Polyvinylacetat-Polyethylenglykol-Pfropfcopolymer, Hydroxypropylcellulose und Hydroxypropylmethylcellulosen, und
das oberflächenaktiven Mittel Polyoxyethylen-Sorbitan-Monooleat, Polyoxyethylen-Sorbitan-Monostearat oder Polyoxyethylen-Sorbitan-Monopalmitat ist.

## Revendications

1. Dispersion solide, qui comprend :
(I) au moins un composé acide peu soluble dans l'eau (API) avec au moins un polymère soluble dans l'eau pharmaceutiquement acceptable, au moins un polymère gastrique soluble et au moins un tensioactif pharmaceutiquement acceptable,
dans laquelle, en l'absence de ladite dispersion solide, le composé acide peu soluble dans l'eau a au moins un pKa (acide) compris dans la plage de 0,0-10,0, et une solubilité dépendante du pH dans un environnement aqueux de pH 1,0-8,0, avec la solubilité aqueuse la plus faible inférieure à 1,0 mg/mL à un pH de 1,0-2,0,
dans laquelle, dans ladite dispersion solide, ledit ou lesdits polymère(s) gastrique soluble(s) est dispersé dans ladite matrice de dispersion solide avec API, polymère(s) hydrosoluble(s) et/ou tensioactif(s) à l'état solide,
dans laquelle, dans ladite dispersion solide, ledit ou lesdits polymère(s) et/ou tensioactif(s) sont présents en une quantité telle que la concentration dudit composé acide peu soluble dans l'eau dissous à la fois à un pH de 1,0-2,0 et à un pH de 6,0-8,0 à ou après 90 minutes lors d'un test de dissolution sans enfoncement dans un environnement aqueux, est au moins deux fois supérieure à la solubilité dudit API seul dans un environnement aqueux à un pH de 1,0-2,0 ne contenant pas ladite dispersion solide,
dans laquelle, dans ladite dispersion solide, ledit ou lesdits polymère(s) et/ou tensioactif(s) sont présents en une quantité telle qu'au bout de 90 minutes ou plus après un test de dissolution sans enfoncement dans un milieu aqueux, le rapport entre la concentration dudit composé acide peu soluble dans l'eau dissous à un pH de 6,0-8,0 et celle à un pH de 1,0-2,0 est inférieur à 2,0; ou
(II) au moins un composé neutre ou non ionisable peu soluble dans l'eau (API) avec au moins un polymère soluble dans l'eau pharmaceutiquement acceptable, au moins un polymère sensible au pH et au moins un tensioactif pharmaceutiquement acceptable,
dans lequel, en l'absence de ladite dispersion solide, le composé neutre ou non ionisable peu soluble dans l'eau n'a pas de pKa détectable (ou calculé) dans la plage de 1,0 à 12,0, et a une solubilité indépendante du pH dans un environnement aqueux de pH 1,0 à 8,0 avec une solubilité aqueuse la plus faible inférieure à 1,0 mg/mL,
dans laquelle, dans ladite dispersion solide, ledit ou lesdits polymère(s) sensible(s) au pH sont disposés dans ladite matrice de dispersion solide avec l'API, le ou les polymère(s) hydrosoluble(s) et/ou le ou les tensioactif(s) à l'état solide,
dans laquelle, dans ladite dispersion solide, ledit ou lesdits polymère(s) et/ou tensioactif(s) sont présents en une quantité telle que la concentration dudit composé dissous peu soluble dans l'eau à la fois à un pH de 1,0-2,0 et pH 6,0-8,0 à ou après 90 minutes lors d'un test de dissolution sans enfoncement dans un environnement aqueux est au moins deux fois supérieure à la solubilité dudit API seul dans un environnement aqueux à un pH de 1,0-8,0 ne contenant pas ladite dispersion solide,
dans laquelle, dans ladite dispersion solide, ledit ou lesdits polymère(s) et/ou tensioactif(s) sont présents en une quantité telle qu'au bout de 90 minutes après un test de dissolution sans enfoncement dans un milieu aqueux, le rapport entre la concentration dudit composé peu soluble dans l'eau dissous à un pH de 6,0-8,0 et celle à un pH de 1,0-2,0 est compris entre 0,5-2,0; et
dans laquelle, dans (I) et (II), le rapport pondéral dudit ou desdits polymère(s) hydrosoluble(s) et dudit ou desdits tensioactif(s) pharmaceutiquement acceptable(s) par rapport audit ou auxdits polymères sensibles au pH ou audit ou auxdits polymère(s) gastrique soluble(s) est compris dans la plage de 0,5:9,5 à 9,5:0,5.

2. La dispersion solide selon la revendication 1, dans laquelle le rapport pondéral dudit ou desdits polymère(s) hydrosoluble(s) et du ou des tensioactif(s) pharmaceutiquement acceptable(s) par rapport audit ou auxdits polymère(s) sensible(s) au pH ou polymère(s) gastrique soluble(s) est compris dans une plage de 1:9 à 1:1, de 1:1 à 9:1 et de 1:2 à 5:1.

3. La dispersion solide selon la revendication 1, dans laquelle ledit ou lesdits polymère(s) et/ou tensioactif(s) sont présents en une quantité telle qu'au bout de 90 minutes après dissolution dans un environnement aqueux non réactif, le rapport entre la concentration d'un composé basique peu soluble dans l'eau dissous à un pH de 1,0-2,0 et celle à un pH de 6,0-8,0 est inférieur à 1,5, et éventuellement inférieur à 1,25.

4. La dispersion solide selon la revendication 1, dans laquelle ledit agent gastrique soluble est choisi dans le groupe constitué par: les copolymères d'acide méthacrylique, en particulier le copolymère de butylméthacrylate-(2-diméthylaminoéthyl)-méthacrylate-méthacrylate de méthyle (1:2:1), qui est un copolymère à base de (2-diméthylaminoéthyl) méthacrylate, de méthacrylate de butyle et de méthacrylate de méthyle ayant un poids moléculaire moyen d'environ 150 000, le chitosane (polysaccharide linéaire composé de D-glucosamine (unité désacétylée) liée de manière aléatoire par des liaisons β-(1-4), et de N-acétyl-D-glucosamine (unité acétylée)), ou tout autre polymère de poids moléculaire élevé comportant un groupe fonctionnel cationique, et toute combinaison de ceux-ci.

5. La dispersion solide selon la revendication 1, dans laquelle la charge en principe actif dudit composé peu soluble dans l'eau est comprise entre 0 % p/p et 95 % p/p.

6. La dispersion solide selon la revendication 1, dans laquelle le rapport pondéral entre le au moins un tensioactif pharmaceutiquement acceptable et le au moins un polymère hydrosoluble pharmaceutiquement acceptable est compris dans une plage allant d'environ 0,01:9,99 à environ 9,99:0,01.

7. La dispersion solide selon la revendication 1, dans laquelle lesdites compositions pharmaceutiques de ladite dispersion sont préparées de la manière suivante:
mélange mécanique dudit API cristallin d'un diamètre inférieur à 10 microns avec un mélange de deux ou plusieurs desdits polymères et/ou dudit tensioactif préparés sous forme de dispersion solide, en particulier par séchage par atomisation, extrusion à chaud ou lyophilisation;
mélange mécanique dudit API amorphe d'un diamètre inférieur à 10 microns avec un mélange de deux ou plusieurs desdits polymères et/ou dudit tensioactif préparé sous forme de dispersion solide, en particulier par séchage par atomisation, extrusion à chaud ou lyophilisation;
dispersion dudit API, à l'état cristallin ou amorphe, avec un diamètre inférieur à dix microns dans une matrice solide desdits deux ou plusieurs polymères et/ou dudit mélange de tensioactifs; ou
dispersion dudit API, à l'état cristallin ou amorphe, avec un diamètre inférieur à dix microns dans une matrice solide desdits deux ou plusieurs polymères, ledit tensioactif étant ajouté en tant que phase externe.

8. La dispersion solide selon la revendication 1, dans laquelle le au moins un polymère sensible au pH est choisi dans le groupe constitué par : les copolymères d'acide méthacrylique, en particulier le copolymère de butylméthacrylate-(2-diméthylaminoéthyl)-méthacrylate-méthacrylate de méthyle (1:2:1), qui est un copolymère à base de (2-diméthylaminoéthyl)méthacrylate, butylméthacrylate et méthacrylate de méthyle ayant un poids moléculaire moyen d'environ 150 000), le chitosane (polysaccharide linéaire composé de D-glucosamine (unité désacétylée) et de N-acétyl-D-glucosamine (unité acétylée) liées de manière aléatoire par des liaisons β-(1-4)), ou d'autres polymères de poids moléculaire élevé avec un groupe fonctionnel cationique, acétate de cellulose phtalate (CAP), hydroxypropylméthylcellulose phtalate (HPMCP-50 ou HPMCP-55), copolymères acryliques solubles dans les alcalis, polyvinylacétate phtalate (PVAP), alginates, carboxyméthylcellulose (CMC) et mélanges d'un ou plusieurs de ceux-ci.

9. La dispersion solide selon la revendication 1, dans laquelle le au moins un polymère hydrosoluble pharmaceutiquement acceptable est choisi dans le groupe constitué par : les homopolymères et copolymères de N-vinyl lactames, en particulier les homopolymères et copolymères de N-vinyl pyrrolidone, par exemple la polyvinylpyrrolidone (PVP), copolymères de N-vinylpyrrolidone et d'acétate de vinyle ou de propionate de vinyle, copolymère greffé de polyvinylcaprolactame-polyvinylacétate-polyéthylèneglycol commercialisé sous forme de copolymères séquencés d'oxyde d'éthylène et d'oxyde de propylène, également connus sous le nom de copolymères séquencés de polyoxyéthylène et de polyoxypropylène ou polyoxyéthylène polypropylèneglycol, tels que le poloxamère, lauroyl polyoxyglycérides esters de cellulose et éthers de cellulose; en particulier la méthylcellulose, les hydroxyalkylcelluloses, en particulier l'hydroxypropylcellulose, les hydroxyalkylalkylcelluloses, en particulier l'hydroxypropylméthylcellulose, les polyalkylène oxydes de haut poids moléculaire tels que le polyéthylène oxyde et le polypropylène oxyde et les copolymères d'oxyde d'éthylène et d'oxyde de propylène, polymères d'acétate de vinyle tels que les copolymères d'acétate de vinyle et d'acide crotonique, le polyvinylacétate partiellement hydrolysé (également appelé « alcool polyvinylique » partiellement saponifié), l'alcool polyvinylique, les oligo- et polysaccharides tels que les carraghénanes, les galactomannanes et la gomme xanthane, et des mélanges d'un ou plusieurs de ceux-ci.

10. La dispersion solide selon la revendication 1,
dans laquelle la dispersion solide comprend au moins un tensioactif pharmaceutiquement acceptable, et
dans laquelle ledit au moins un tensioactif pharmaceutiquement acceptable est choisi dans le groupe constitué par : les éthers d'alkyle de polyoxyéthylène, les éthers d'alkylaryle de polyoxyéthylène, les esters d'acides gras de polyéthylène glycol, les monoesters d'acides gras d'alkylène glycol, les esters d'acides gras de saccharose, les monoesters d'acides gras de sorbitane, les polyoxyglycérides de lauroyle, les copolymères greffés de polyvinylcaprolactame-polyvinylacétate-polyéthylèneglycol commercialisés sous le nom de docusate sodique, polyéthylène glycol-26 glycérine, monostéarate de polyoxyéthylène, succinate de d-α-tocophéryle polyéthylène glycol 1000 (vitamine E TPGS), éthers d'alkyle de polyoxyéthylène, esters d'acides gras de polyéthylène glycol, monoesters d'acides gras d'alkylène glycol, esters d'acides gras de saccharose, monoesters d'acides gras de sorbitane, stéarate de sorbitane, dérivés de polyoxyéthylène et d'huile de ricin, ou oxystéarate de polyoxyéthylène glycérol ou copolymères blocs d'oxyde d'éthylène et d'oxyde de propylène, également connus sous le nom de copolymères blocs polyoxyéthylène polyoxypropylène ou polyoxyéthylène polypropylène glycol, tels que le poloxamère ou un ester monoacide gras de polyoxyéthylène sorbitane, par exemple le mono-oléate de polyoxyéthylène sorbitane, et des mélanges d'un ou plusieurs de ceux-ci.

11. Procédé de préparation d'une forme posologique pharmaceutique destinée à être administrée par voie orale à un mammifère à l'aide de ladite dispersion solide selon la revendication 1.

12. La dispersion solide selon la revendication 1, dans laquelle l'API est le composé acide soluble dans l'eau.

13. La dispersion solide selon la revendication 1, dans laquelle l'API est le composé neutre ou non ionisable peu soluble dans l'eau.

14. La dispersion solide selon la revendication 3, dans laquelle au moins l'un desdits composés basiques peu solubles dans l'eau (API) est Prasugrel ou Clopidogrel;
dans laquelle, en l'absence de ladite dispersion solide, le composé basique peu soluble dans l'eau a au moins un pKa (base) compris dans la plage de 0,0-10,0, et une solubilité dépendante du pH dans un environnement aqueux de pH 1,0-8,0 avec la solubilité aqueuse la plus faible < 1,0 mg/mL à pH 6,0-8,0;
dans laquelle, dans ladite dispersion solide, ledit ou lesdits polymère(s) gastrique soluble(s) sont dispersés dans une matrice de dispersion solide avec un API, un ou plusieurs polymères hydrosolubles et/ou un ou plusieurs tensioactifs à l'état solide;
dans laquelle le polymère gastrique soluble est un succinate d'acétate d'hydroxypropylméthylcellulose;
dans laquelle ledit polymère hydrosoluble est choisi dans le groupe constitué par le copolymère greffé polyvinylcaprolactame-polyvinylacétate-polyéthylèneglycol, l'hydroxypropylcellulose et les hydroxypropylméthylcelluloses, et
ledit tensioactif est le mono-oléate de polyoxyéthylène sorbitane, le monostéarate de polyoxyéthylène sorbitane ou le monopalmitate de polyoxyéthylène sorbitane.

15. La dispersion solide selon la revendication 1, dans laquelle au moins un desdits composés acides peu solubles dans l'eau (API) est choisi dans le groupe constitué par le diclofénac ou l'ibuprofène;
dans laquelle, en l'absence de ladite dispersion solide, le composé acide peu soluble dans l'eau a au moins un pKa (acide) compris dans la plage de 0,0-10,0, et une solubilité dépendante du pH dans un environnement aqueux de pH 1,0-8,0 avec la solubilité aqueuse la plus faible < 1,0 mg/mL à pH 1,0-2,0;
dans laquelle, dans ladite dispersion solide, ledit ou lesdits polymères gastrosolubles sont dispersés dans une matrice de dispersion solide avec un API, un ou plusieurs polymères hydrosolubles et/ou un ou plusieurs tensioactifs à l'état solide;
dans laquelle le polymère gastrosoluble est un succinate d'acétate d'hydroxypropylméthylcellulose;
dans laquelle ledit polymère hydrosoluble est choisi dans le groupe constitué par le copolymère greffé polyvinylcaprolactame-polyvinylacétate-polyéthylèneglycol, l'hydroxypropylcellulose et les hydroxypropylméthylcelluloses, et
ledit tensioactif est le mono-oléate de polyoxyéthylène sorbitane, le monostéarate de polyoxyéthylène sorbitane ou le monopalmitate de polyoxyéthylène sorbitane.
